# EUROPEAN PATENT SPECIFICATION

(11) **EP 3 681 901 B1**
(45) Date of publication and mention of the grant of the patent: **27.03.2024**
(21) Application number: 18769331.2
(22) Date of filing: 11.09.2018
(51) Int. Cl.: C07K 14/005, C12N 5/074

(54) **METHOD OF ENHANCING RNA EXPRESSION IN A CELL**
VERFAHREN ZUR VERBESSERUNG DER RNA-EXPRESSION IN EINER ZELLE
PROCEDE D'AMÉLIORATION DE L'EXPRESSION DE L'ARN DANS UNE CELLULE

(30) Priority: 13.09.2017 WO PCT/EP2017/073065
(43) Date of publication of application: 22.07.2020
(73) Proprietor: BioNTech SE, 55131 Mainz (DE); TRON - Translationale Onkologie an der Universitätsmedizin der Johannes Gutenberg- Universität Mainz gemeinnützige GmbH, 55131 Mainz (DE)
(72) Inventor: POLEGANOV, Marco Alexander, 55131 Mainz (DE); PERKOVIC, Mario, 55131 Mainz (DE); SAHIN, Ugur, 55131 Mainz (DE); BEISSERT, Tim, 55131 Mainz (DE); KUHN, Andreas, 55131 Mainz (DE)
(74) Representative: Schnappauf, Georg
(86) International application number: PCT/EP2018/074449
(87) International publication number: WO 2019/053003

(56) References cited:
- EP-A1- 2 917 350
- MARCO ALEXANDER POLEGANOV ET AL: "Efficient Reprogramming of Human Fibroblasts and Blood-Derived Endothelial Progenitor Cells Using Nonmodified RNA for Reprogramming and Immune Evasion", HUMAN GENE THERAPY, vol. 26, no. 11, 1 November 2015 (2015-11-01), pages 751-766, XP055239636, US ISSN: 1043-0342, DOI: 10.1089/hum.2015.045
- G. GORI-SAVELLINI ET AL: "Toscana Virus NSs Protein Inhibits the Induction of Type I Interferon by Interacting with RIG-I", JOURNAL OF VIROLOGY., vol. 87, no. 12, 15 June 2013 (2013-06-15) , pages 6660-6667, XP055515293, US ISSN: 0022-538X, DOI: 10.1128/JVI.03129-12
- F. WEBER ET AL: "Bunyamwera Bunyavirus Nonstructural Protein NSs Counteracts the Induction of Alpha/Beta Interferon", JOURNAL OF VIROLOGY, vol. 76, no. 16, 15 August 2002 (2002-08-15), pages 7949-7955, XP055076555, ISSN: 0022-538X, DOI: 10.1128/JVI.76.16.7949-7955.2002

## Description

### TECHNICAL FIELD

The present teaching describes a virus-derived factor which when provided to cells, e.g., by transfecting the cells with RNA encoding the virus-derived factor, enhances expression of RNA encoding a peptide or protein in the cells. In particular, the virus-derived factor enhances survival of cells, in particular when transfected repetitively with RNA, and reduces an IFN response of cells to transfected RNA. Accordingly, the present teaching provides methods and means for enhancing expression of RNA in cells. The cells are preferably transfected with the RNA.

### BACKGROUND

The use of RNA for delivery of foreign genetic information into target cells offers an attractive alternative to DNA. The advantages of using RNA include transient expression and a non-transforming character. RNA does not need to enter the nucleus in order to be expressed and moreover cannot integrate into the host genome, thereby eliminating the risk of oncogenesis.

RNA has been described as being useful in de-differentiating somatic cells into stem-like cells without generating embryos or fetuses. De-differentiation of somatic cells to cells having stem cell characteristics, in particular pluripotency, can be effected by introducing RNA encoding factors inducing the de-differentiation of somatic cells into the somatic cells (also termed reprogramming transcription factors (rTF) or simply reprogramming factors) and culturing the somatic cells allowing the cells to de-differentiate. After being de-differentiated, the cells can be induced to re-differentiate into the same or a different somatic cell type such as neuronal, hematopoietic, muscle, epithelial, and other cell types. Thus, such stem-like cells have medical applications for treatment of degenerative diseases by "cell therapy" and may be utilized in novel therapeutic strategies in the treatment of cardiac, neurological, endocrinological, vascular, retinal, dermatological, muscular-skeletal disorders, and other diseases.

Weber et al. (Journal of Virology, 2002, 76(16):7949-7955) disclose that the nonstructural protein NSs of Bunyamwera virus, a member of the *Bunyaviridae* family, counteracts the induction of alpha/beta Interferon. Gori-Savellini et al. (Journal of Virology 2013, 87(12):6660-6667) describe that the NSs protein of Toscana virus, also a member of the *Bunyaviridae* family, inhibits the induction of type I interferon by interacting with RIG-I.

WO 2014/072061 A1 relates to expressing RNA in cells and to enhancing viability of cells in which RNA is to be expressed. The methods for expressing RNA in cells comprise steps of preventing engagement of IFN receptor by extracellular IFN and inhibiting intracellular IFN signalling in the cells. This allows repetitive transfer of RNA into the cells.

Poleganov et al. (Human Gene Therapy 2015, 26(11):751-766) describe reprogramming of human fibroblasts and blood-derived endothelial progenitor cells using nonmodified RNA for reprogramming and immune evasion.

However, exogenous single-stranded RNA activates defense mechanisms in mammalian cells. Thus, RNA-based gene transfer generally is accompanied with an induction of an IFN-response which hinders the continuous expression of the delivered RNA.

### Summary of the invention

The present invention is defined by the appended claims.

In one aspect, the present invention relates to an *in vitro* method for expressing one or more chains of a T cell receptor or of an artificial T cell receptor in a T-cell or in an antigen presenting cell, the method comprising the steps of
(i) introducing RNA encoding one or more chains of a T cell receptor or of an artificial T cell receptor into the cell and
(ii) providing *Toscana virus* NSs protein to the cell,
wherein providing *Toscana virus* NSs protein to the cell comprises introducing RNA encoding the *Toscana virus* NSs protein into the cell.

In one embodiment, the RNA is introduced into the cell repetitively. In some embodiments, the RNA is introduced into the cell by electroporation or lipofection. In some embodiments, the RNA is *in vitro* transcribed RNA.

In some embodiments, the method further comprises providing vaccinia virus B18R and/or vaccinia virus E3 to the cell, wherein providing vaccinia virus B18R and/or vaccinia virus E3 to the cell comprises introducing RNA encoding vaccinia virus B18R and/or RNA encoding vaccinia virus E3 into the cell.

In some embodiments, the cell is a human cell.

In a further aspect, the present invention relates to an *in vitro* method of producing immunoreactive cells comprising the step of
(i) introducing into a T cell or a progenitor thereof one or more RNA molecules encoding the chains of a T cell receptor or the chain(s) of an artificial T cell receptor, and
(ii) providing Toscana virus NSs protein,
wherein providing *Toscana virus* NSs protein to the cell comprises introducing RNA encoding the *Toscana virus* NSs protein into the cell.

In one embodiment the immunoreactive cells comprise T cells such as cytotoxic T cells, helper T cells, or tumor infiltrating T cells. In some embodiments, the immunoreactive cells comprise CD4+ and/or CD8+ T cells. In a preferred embodiment, the immunoreactive cells comprise cells which can mature into immune cells, such as T cell, in particular T helper cell, or cytolytic T cell, with suitable stimulation. In a further preferred embodiment, the immunoreactive cells comprise CD34+ hematopoietic stem cells, immature and mature T cells.

In some embodiments, the T cell receptor comprises α- and β-TCR chains. In some embodiments, the artificial T cell receptor comprises an antigen binding domain such as antigen-binding portions of antibodies and T cell receptors such as single-chain variable fragments (scFv), a T cell signaling domain, such as the endodomain of CD3-zeta, and optionally one or more co-stimulation domains, such as CD28, CD137 (4-1 BB), CD134 (OX40), and CD278 (ICOS).

### Summary of the teaching

The present disclosure provides teachings which, in some respects, go beyond the invention as such, which is defined exclusively by the appended claims. The teachings are provided to place the actual invention in a broader technical context and to illustrate possible related technical developments. Such additional technical information which does not fall within the scope of the appended claims, is not part of the invention. In particular, the terms "embodiment" and "aspect" are not to be construed as necessarily referring to an embodiment of the invention, unless the "embodiment" or "aspect" in question falls within the scope of the claims.

It has been observed according to the teaching that the viral escape protein NSs (N) from *Toscana virus* is a potent inhibitor of IFN-response and can be used in combination with RNA transfection. Furthermore, it has been observed according to the teaching that the viral escape protein NSs (N) from *Toscana virus* can be used to substitute EKB (E3, K3, B18R) for successful RNA-based expression of proteins of interest.

In one aspect, the teaching relates to a method for expressing a peptide or protein (also termed peptide or protein of interest herein) in a cell comprising the steps of (i) introducing RNA encoding the peptide or protein into the cell and (ii) providing a virus-derived factor comprising *Toscana virus* NSs protein or a functional variant of *Toscana virus* NSs protein to the cell.

In one embodiment, the virus-derived factor comprises *Toscana virus* NSs protein.

In one embodiment, the RNA is introduced into the cell repetitively. In one embodiment, the RNA is introduced into the cell by electroporation or lipofection.

In one embodiment, the RNA is *in vitro* transcribed RNA.

In one embodiment, providing the virus-derived factor to the cell comprises introducing nucleic acid such as RNA encoding the virus-derived factor into the cell.

In one embodiment, the method of the teaching may additionally comprise (i) preventing engagement of IFN receptor by extracellular IFN and/or (ii) inhibiting intracellular IFN signalling. Thus, in one embodiment, the method of the teaching comprises providing one or more of vaccinia virus B18R, vaccinia virus E3 and vaccinia virus K3 such as vaccinia virus B18R and/or vaccinia virus E3 to the cell. In one embodiment, providing one or more of vaccinia virus B18R, vaccinia virus E3 and vaccinia virus K3 to the cell comprises introducing nucleic acid such as RNA encoding the same into the cell.

However, it has been observed according to the teaching that the viral escape protein NSs (N) from *Toscana virus* can be used to completely substitute EKB (E3, K3, B18R) for successful RNA-based expression of proteins of interest. Thus, in one embodiment, the method of the teaching comprises providing the virus-derived factor to the cell but does not comprise providing one or more of vaccinia virus B18R, vaccinia virus E3 and vaccinia virus K3 to the cell, preferably does not comprise providing any of vaccinia virus B18R, vaccinia virus E3 and vaccinia virus K3 to the cell, and more preferably does not comprise providing any inhibitor of the IFN response except the virus-derived factor to the cell.

In one embodiment, providing the virus-derived factor to the cell enhances stability and/or expression of the RNA in the cell compared to the situation where the virus-derived factor is not provided.

In one embodiment, the enhancement of expression of the RNA in the cell preferably comprises an increase in the level of expression and/or an increase in the duration of expression of the RNA in the cell.

In one embodiment, providing the virus-derived factor to the cell enhances cell viability compared to the situation where the virus-derived factor is not provided.

In one embodiment, the cell is a cell having a barrier function. In one embodiment, the cell is a fibroblast, a keratinocyte, an epithelial cell, or an endothelial cell, wherein the endothelial cell preferably is an endothelial cell of the heart, an endothelial cell of the lung, or an umbilical vein endothelial cell. In one embodiment, the cell is a T cell or an antigen presenting cell. In one embodiment, the cell is a human cell.

The teaching also relates to a method for providing cells having stem cell characteristics comprising the steps of (i) providing a cell population comprising somatic cells, (ii) providing a virus-derived factor comprising *Toscana virus* NSs protein or a functional variant of *Toscana virus* NSs protein to the somatic cells, (iii) introducing RNA encoding one or more reprogramming factors into the somatic cells, and (iv) allowing the development of cells having stem cell characteristics.

In one embodiment, the virus-derived factor comprises *Toscana virus* NSs protein.

In one embodiment, the RNA is introduced into the somatic cells repetitively. In one embodiment, the RNA is introduced into the somatic cells by electroporation or lipofection.

In one embodiment, the RNA is *in vitro* transcribed RNA.

In one embodiment, providing the virus-derived factor to the somatic cells comprises introducing nucleic acid such as RNA encoding the virus-derived factor into the somatic cells.

In one embodiment, the method of the teaching may additionally comprise (i) preventing engagement of IFN receptor by extracellular IFN and/or (ii) inhibiting intracellular IFN signalling. Thus, in one embodiment, the method of the teaching comprises providing one or more of vaccinia virus B18R, vaccinia virus E3 and vaccinia virus K3 such as vaccinia virus B18R and/or vaccinia virus E3 to the somatic cells. In one embodiment, providing one or more of vaccinia virus B18R, vaccinia virus E3 and vaccinia virus K3 to the somatic cells comprises introducing nucleic acid such as RNA encoding the same into the somatic cells.

However, it has been observed according to the teaching that the viral escape protein NSs (N) from *Toscana virus* can be used to completely substitute EKB (E3, K3, B18R) for successful RNA-based expression of proteins of interest. Thus, in one embodiment, the method of the teaching comprises providing the virus-derived factor to the somatic cells but does not comprise providing one or more of vaccinia virus B18R, vaccinia virus E3 and vaccinia virus K3 to the somatic cells, preferably does not comprise providing any of vaccinia virus B18R, vaccinia virus E3 and vaccinia virus K3 to the somatic cells, and more preferably does not comprise providing any inhibitor of the IFN response except the virus-derived factor to the somatic cells.

In one embodiment, providing the virus-derived factor to the somatic cells enhances stability and/or expression of the RNA in the somatic cells compared to the situation where the virus-derived factor is not provided.

In one embodiment, the enhancement of expression of the RNA in the somatic cells preferably comprises an increase in the level of expression and/or an increase in the duration of expression of the RNA in the somatic cells.

In one embodiment, providing the virus-derived factor to the somatic cells enhances cell viability compared to the situation where the virus-derived factor is not provided.

In one embodiment, the method further comprises introducing into the somatic cells miRNA enhancing reprogramming of the somatic cells to cells having stem cell characteristics.

In one embodiment, the one or more reprogramming factors comprise OCT4 and SOX2. The one or more reprogramming factors may further comprise KLF4 and/or c-MYC and/or NANOG and/or LIN28. In one embodiment, the one or more reprogramming factors comprise OCT4, SOX2, KLF4 and c-MYC and may further comprise LIN28 and optionally NANOG. In one embodiment, the one or more reprogramming factors comprise OCT4, SOX2, NANOG and LIN28.

In one embodiment, the method further comprises the step of culturing the somatic cells in the presence of at least one histone deacetylase inhibitor, wherein the at least one histone deacetylase inhibitor preferably comprises valproic acid, sodium butyrate, trichostatin A and/or scriptaid.

In one embodiment, the step of allowing the development of cells having stem cell characteristics comprises culturing the somatic cells under embryonic stem cell culture conditions.

In one embodiment, the stem cell characteristics comprise an embryonic stem cell morphology.

In one embodiment, the cells having stem cell characteristics have normal karyotypes, express telomerase activity, express cell surface markers that are characteristic for embryonic stem cells and/or express genes that are characteristic for embryonic stem cells.

In one embodiment, the cells having stem cell characteristics exhibit a pluripotent state.

In one embodiment, the cells having stem cell characteristics have the developmental potential to differentiate into advanced derivatives of all three primary germ layers.

In one embodiment, the somatic cells are fibroblasts such as lung fibroblasts, foreskin fibroblasts or dermal fibroblasts, keratinocytes or endothelial progenitor cells. Preferably, the somatic cells are human cells.

The teaching also relates to a method for providing differentiated cell types comprising the steps of (i) providing cells having stem cell characteristics using the method for providing cells having stem cell characteristics according to the teaching, and (ii) culturing the cells having stem cell characteristics under conditions that induce or direct partial or complete differentiation to a differentiated cell type.

The teaching also relates to a composition comprising nucleic acid such as RNA encoding a virus-derived factor comprising *Toscana virus* NSs protein or a functional variant of *Toscana virus* NSs protein. Furthermore, the teaching also relates to a kit comprising the composition of the teaching. In one embodiment, the composition or kit of the teaching is useful in the methods of the teaching. Accordingly, the kit may comprise instructions such as printed instructions relating to the use of the kit in the methods described herein. Various embodiments of the composition or kit of the teaching are described above for the methods of the teaching.

In one embodiment, the composition or kit of the teaching comprises RNA to be introduced into a cell for expression, e.g., RNA encoding one or more peptides or proteins of interest such as one or more reprogramming factors.

The teaching may be utilized, for example, for increasing expression of peptides or proteins of interest upon transfection of cells with RNA encoding such peptides or proteins of interest. More specifically, it is possible, when producing recombinant proteins, to use the methods and agents of the teaching for expression of recombinant proteins in cell-based systems. This allows inter alia production costs to be reduced.

It is also possible to use the methods and agents of the teaching for gene therapy applications. Accordingly, RNA to be transfected may be a gene therapy vector and may be used for expression of a transgene. Cells can be transfected with these vectors *in vitro,* for example in lymphocytes or dendritic cells, or else *in vivo* by direct administration.

RNA may be employed, for example, for transient expression of genes, with possible fields of application being RNA-based vaccines which are transfected into cells *in vitro* or administered directly *in vivo,* transient expression of functional recombinant proteins *in vitro,* for example in order to initiate differentiation processes in cells or to study functions of proteins, and transient expression of functional recombinant proteins such as erythropoietin, hormones, coagulation inhibitors, etc., *in vivo,* in particular as pharmaceuticals.

RNA may be used in particular for transfecting antigen-presenting cells and thus as a tool for delivering the antigen to be presented and for loading antigen-presenting cells with said antigen to be presented *in vitro* and *in vivo.* Such antigen-presenting cells may be used for stimulating T cells, in particular CD4⁺ and/or CD8⁺ T cells.

### Detailed description

The present teaching is described in detail below. The terminology used herein is for the purpose of describing particular embodiments. Unless defined otherwise, all technical and scientific terms used herein have the same meanings as commonly understood by one of ordinary skill in the art.

In the following, the elements of the present teaching will be described. These elements are listed with specific embodiments, however, it should be understood that they may be combined in any manner and in any number to create additional embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

The practice of the present teaching will employ, unless otherwise indicated, conventional methods of chemistry, biochemistry, cell biology, immunology, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The terms "a" and "an" and "the" and similar reference used in the context of describing the teaching (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as"), provided herein is intended to better illustrate the teaching.

It has been observed according to the teaching that the viral escape protein NSs (N) from *Toscana virus* is a potent inhibitor of IFN-response and can be used in combination with RNA transfection in order to enhance expression of a peptide or protein of interest encoded by the RNA.

In one embodiment, a method of the teaching is an *in vitro* method. In one embodiment, a method of the teaching comprises or does not comprise the removal of cells from a human or animal subject, e.g., by surgery. The cells resulting from the methods of the teaching may be administered to a subject. The cells may be autologous, syngenic, allogenic or heterologous with respect to the subject. Transfected cells may be introduced into a subject using any means known in the art. In other embodiments, the cells may be present in a subject, such as a patient. In these embodiments, the methods of the teaching are *in vivo* methods which comprise administration of RNA to the subject.

In one embodiment of the teaching, cells are provided with a virus-derived factor comprising *Toscana virus* NSs protein or a functional variant of *Toscana virus* NSs protein prior to, simultaneously with and/or following introduction of RNA encoding the peptide or protein to be expressed, e.g., one or more reprogramming factors, or the first introduction (e.g. in case of repeated transfections) of RNA. In one embodiment, cells are provided with a virus-derived factor comprising *Toscana virus* NSs protein or a functional variant of *Toscana virus* NSs protein following, preferably immediately following introduction of RNA or the first introduction (e.g. in case of repeated transfections) of RNA.

In one particularly preferred embodiment, cells are provided with a virus-derived factor comprising *Toscana virus* NSs protein or a functional variant of *Toscana virus* NSs protein by introducing RNA encoding the virus-derived factor into the cells. In one embodiment of the teaching, RNA encoding the virus-derived factor is introduced into the cells prior to, simultaneously with and/or following introduction of RNA encoding the peptide or protein to be expressed, or the first introduction (e.g. in case of repeated transfections) of RNA encoding the peptide or protein to be expressed. In one embodiment, RNA encoding the virus-derived factor is introduced into the cells following, preferably immediately following introduction of RNA encoding the peptide or protein to be expressed or the first introduction (e.g. in case of repeated transfections) of RNA encoding the peptide or protein to be expressed.

The family Bunyaviridae includes the animal-infecting genera Orthobunyavirus, Phlebovirus, Hantavirus, and Nairovirus and the plant-infecting genus Tospovirus. Phleboviruses are comprised of the sandfly fever group and the Uukuniemi group. Several members of the sandfly fever group including Rift Valley fever virus (RVFV), Toscana virus (TOSV), Sandfly fever Sicilian virus (SFSV), and Punta toro virus (PTV) are pathogenic to humans. TOSV is an enveloped virus carrying a negative-sense, single-stranded RNA genome comprising three segments, which are designated L (large), M (medium), and S (small). The L-segment encodes the viral polymerase (L), the M-segment encodes the envelope glycoproteins Gn and Gc, and the S-segment encodes the nucleoprotein (N). In addition, the S-segment also encodes the nonstructural protein NSs in an ambisense manner. Phlebovirus NSs proteins play an important role in viral evasion from host innate immune responses.

According to the teaching, the term "Toscana virus NSs protein" relates to the nonstructural protein NSs from Toscana virus. In particular, the term relates to a protein comprising the following amino acid sequence:

MQSRAVILKHRSGSGHKRSLPRFYIDCDLDTFDFEKGCSLIENEFPIYINNYEVVYKS
KPTLSHFLIEKEFPAVLGPGMISAVRTRLYEPTMRELYQESIHQLKRNNKKYLLSALR
WPTGIPTLEFIDYYFEELLFLSEFDPGSIQRYLKLLVKASGLYNSTIEEQLVEIHRRVLI
EGKKHGLTAFDLPGNDILGDICVVQAARVTRLVAKTFSKMTRDTHLMIYFSISPVELV
LNKLDKKEDKRAKAKGLMSMCAARSYDYFMRTDLGFRETALSTFWAKDWPTLQET
ILSDKRCLKEDMRVTKWLPSPPHYPPL (SEQ ID NO: 1).

The term "functional variant of *Toscana virus* NSs protein" relates to any protein, in particular any virus-derived protein exhibiting functions similar to *Toscana virus* NSs protein. One particular function is to enhance expression of RNA encoding a peptide or protein in cells. Accordingly, the term includes proteins comprising functional fragments of *Toscana virus* NSs protein or functional sequence variants of *Toscana virus* NSs protein or fragments thereof.

"Fragment", with reference to an amino acid sequence (peptide or protein), relates to a part of an amino acid sequence, i.e. a sequence which represents the amino acid sequence shortened at the N-terminus and/or C-terminus. A fragment shortened at the C-terminus (N-terminal fragment) is obtainable e.g. by translation of a truncated open reading frame that lacks the 3'-end of the open reading frame. A fragment shortened at the N-terminus (C-terminal fragment) is obtainable e.g. by translation of a truncated open reading frame that lacks the 5'-end of the open reading frame, as long as the truncated open reading frame comprises a start codon that serves to initiate translation. A fragment of an amino acid sequence comprises e.g. at least 50 %, at least 60 %, at least 70 %, at least 80%, at least 90% of the amino acid residues from an amino acid sequence.

The term "variant" includes all splice variants, posttranslationally modified variants, conformations, isoforms and species homologs, in particular those which are naturally expressed by cells.

For the purposes of the present teaching, "variants" of an amino acid sequence comprise amino acid insertion variants, amino acid addition variants, amino acid deletion variants and/or amino acid substitution variants. Amino acid insertion variants comprise insertions of single or two or more amino acids in a particular amino acid sequence. In the case of amino acid sequence variants having an insertion, one or more amino acid residues are inserted into a particular site in an amino acid sequence, although random insertion with appropriate screening of the resulting product is also possible. Amino acid addition variants comprise amino- and/or carboxy-terminal fusions of one or more amino acids, such as 1, 2, 3, 5, 10, 20, 30, 50, or more amino acids. Amino acid deletion variants are characterized by the removal of one or more amino acids from the sequence, such as by removal of 1, 2, 3, 5, 10, 20, 30, 50, or more amino acids. The deletions may be in any position of the protein. Amino acid deletion variants that comprise the deletion at the N-terminal and/or C-terminal end of the protein are also called N-terminal and/or C-terminal truncation variants. Amino acid substitution variants are characterized by at least one residue in the sequence being removed and another residue being inserted in its place. Preference is given to the modifications being in positions in the amino acid sequence which are not conserved between homologous proteins or peptides and/or to replacing amino acids with other ones having similar properties. Preferably, amino acid changes in protein variants are conservative amino acid changes, i.e., substitutions of similarly charged or uncharged amino acids. A conservative amino acid change involves substitution of one of a family of amino acids which are related in their side chains. Naturally occurring amino acids are generally divided into four families: acidic (aspartate, glutamate), basic (lysine, arginine, histidine), non-polar (alanine, valine, leucine, isoleucine, proline, phenylalanine, methionine, tryptophan), and uncharged polar (glycine, asparagine, glutamine, cysteine, serine, threonine, tyrosine) amino acids. Phenylalanine, tryptophan, and tyrosine are sometimes classified jointly as aromatic amino acids.

Preferably the degree of similarity, preferably identity between a given amino acid sequence and an amino acid sequence which is a variant of said given amino acid sequence will be at least about 60%, 65%, 70%, 80%, 81%, 82%, 83%, 84%, 84%, 85%, 86%, 87%, 88%, 89%, 90%, 91%, 92%, 93%, 94%, 95%, 96%, 97%, 98%, or 99%. The degree of similarity or identity is given preferably for an amino acid region which is at least about 10%, at least about 20%, at least about 30%, at least about 40%, at least about 50%, at least about 60%, at least about 70%, at least about 80%, at least about 90% or about 100% of the entire length of the reference amino acid sequence. For example, if the reference amino acid sequence consists of 200 amino acids, the degree of similarity or identity is given preferably for at least about 20, at least about 40, at least about 60, at least about 80, at least about 100, at least about 120, at least about 140, at least about 160, at least about 180, or about 200 amino acids, preferably continuous amino acids. In preferred embodiments, the degree of similarity or identity is given for the entire length of the reference amino acid sequence. The alignment for determining sequence similarity, preferably sequence identity can be done with art known tools, preferably using the best sequence alignment, for example, using Align, using standard settings, preferably EMBOSS::needle, Matrix: Blosum62, Gap Open 10.0, Gap Extend 0.5.

"Sequence similarity" indicates the percentage of amino acids that either are identical or that represent conservative amino acid substitutions. "Sequence identity" between two amino acid sequences indicates the percentage of amino acids or nucleotides that are identical between the sequences.

The term "percentage identity" is intended to denote a percentage of amino acid residues which are identical between the two sequences to be compared, obtained after the best alignment, this percentage being purely statistical and the differences between the two sequences being distributed randomly and over their entire length. Sequence comparisons between two amino acid sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Neddleman and Wunsch, 1970, J. Mol. Biol. 48, 443, by means of the similarity search method of Pearson and Lipman, 1988, Proc. Natl Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).

The percentage identity is calculated by determining the number of identical positions between the two sequences being compared, dividing this number by the number of positions compared and multiplying the result obtained by 100 so as to obtain the percentage identity between these two sequences.

Homologous amino acid sequences exhibit according to the teaching at least 40%, in particular at least 50%, at least 60%, at least 70%, at least 80%, at least 90% and preferably at least 95%, at least 98 or at least 99% identity of the amino acid residues.

The amino acid sequence variants described herein may readily be prepared by the skilled person, for example, by recombinant DNA manipulation. The manipulation of DNA sequences for preparing peptides or proteins having substitutions, additions, insertions or deletions, is described in detail in Sambrook et al. (1989), for example. Furthermore, the peptides and amino acid variants described herein may be readily prepared with the aid of known peptide synthesis techniques such as, for example, by solid phase synthesis and similar methods.

The teaching includes derivatives of the peptides or proteins described herein which are comprised by the terms "peptide" and "protein". According to the teaching, "derivatives" of proteins and peptides are modified forms of proteins and peptides. Such modifications include any chemical modification and comprise single or multiple substitutions, deletions and/or additions of any molecules associated with the protein or peptide, such as carbohydrates, lipids and/or proteins or peptides. In one embodiment, "derivatives" of proteins or peptides include those modified analogs resulting from glycosylation, acetylation, phosphorylation, amidation, palmitoylation, myristoylation, isoprenylation, lipidation, alkylation, derivatization, introduction of protective/blocking groups, proteolytic cleavage or binding to an antibody or to another cellular ligand. The term "derivative" also extends to all functional chemical equivalents of said proteins and peptides. Preferably, a modified peptide has increased stability and/or increased immunogenicity.

In one embodiment, the methods of the teaching may additionally comprise (i) preventing engagement of IFN receptor by extracellular IFN and/or (ii) inhibiting intracellular IFN signalling. This can be achieved by providing to a cell (i) an agent preventing engagement of IFN receptor by extracellular IFN and/or (ii) an agent inhibiting intracellular IFN signalling.

In one embodiment, cells are treated to (i) prevent engagement of IFN receptor by extracellular IFN and/or (ii) inhibit intracellular IFN signalling prior to, simultaneously with and/or following introduction of RNA encoding the peptide or protein to be expressed, or the first introduction (e.g. in case of repeated transfections) of RNA. In one embodiment, cells are treated to (i) prevent engagement of IFN receptor by extracellular IFN and/or (ii) inhibit intracellular IFN signalling following, preferably immediately following introduction of RNA or the first introduction (e.g. in case of repeated transfections) of RNA.

Interferons are important cytokines characterized by antiviral, antiproliferative and immunomodulatory activities. The interferons can be grouped into two types. IFN-gamma is the sole type II interferon; all others are type I interferons. Type I and type II interferons differ in gene structure (type II interferon genes have three exons; type I, one), chromosome location (in humans, type II is located on chromosome-12; the type I interferon genes are linked and on chromosome-9), and the types of tissues where they are produced (type I interferons are synthesized ubiquitously, type II by lymphocytes). Type I interferons competitively inhibit each others binding to cellular receptors, while type II interferon has a distinct receptor. According to the teaching, the term "interferon" or "IFN" preferably relates to type I interferons, in particular IFN-alpha and IFN-beta.

IFNs induce the expression of a plethora of antiviral genes, which can interfere with the viral replication cycle. According to the teaching, the term "antivirally active effector protein" relates to a group of proteins encoded by IFN-stimulated genes (ISGs) the transcription of which is signaled by type I IFNs. These proteins target distinct viral components and distinct stages of the viral life cycle, aiming to eliminate invading viruses. "Antivirally active effector proteins" are involved in different effector pathways individually blocking viral transcription, degrading viral RNA, inhibiting translation, and modifying protein function to control all steps of viral replication. Such proteins include 2',5'-oligoadenylate synthetase (OAS), in particular 2',5'-oligoadenylate synthetase 1 (OAS1), RNA-dependent protein kinase R (PKR), and RNaseL. Both PKR and OAS are directly activated by dsRNA. Hence, dsRNA induces the expression of these antivirally active effector proteins and is also necessary for their activation.

According to the teaching, the term "preventing engagement of IFN receptor by extracellular IFN" relates to an inhibition, i.e. blocking, or reduction, of the interaction of IFNs, in particular type I IFNs, with their specific receptors thus, inhibiting or reducing IFN function. Engagement of IFN receptor by extracellular IFN may be prevented, for example, by the provision of a binding agent for extracellular IFN. Those aspects of the present teaching which involve one or more proteins or peptides in preventing engagement of IFN receptor by extracellular IFN, such as proteins or peptides disclosed herein, e.g. one or more binding agents for extracellular IFN, may involve the provision of nucleic acids, in particular RNA, encoding these one or more proteins or peptides to cells, e.g. by introducing the nucleic acids into cells.

For example, the B18R protein is a vaccinia virus-encoded type I interferon receptor with specificity for mouse, human, rabbit, pig, rat, and cow type I interferons which has potent neutralizing activity. The B18R protein encoded by the B18R gene of the Western Reserve vaccinia virus strain. The 60-65 kD glycoprotein is related to the interleukin-1 receptors and is a member of the immunoglobulin superfamily, unlike other type I IFN-receptors, which belong to the class II cytokine receptor family. The B18R protein has a high affinity (KD, 174 pM) for human IFN alpha. Among viral host response modifiers, the B18R protein is unique in that it exists as a soluble extracellular, as well as a cell surface protein, enabling blockage of both autocrine and paracrine IFN functions. The B18R protein has been shown to inhibit the antiviral potency of IFN-alpha1, IFN-alpha2, IFN-alpha-8/1/8, and IFN-omega on human cells. The soluble B18R protein is highly potent for neutralizing type I interferons, which include IFN-alpha, beta, delta, kappa.

Engagement of IFN receptor by extracellular IFN may further be prevented, for example, by reducing the level of IFN, in particular extracellular IFN. In one embodiment, engagement of IFN receptor by extracellular IFN is prevented by interfering with IFN gene expression. For example, Hepatitis C virus serine protease NS3/4A protein complex is able to interfere with and reduce IFN promoter activity and is a specific inhibitor of IFN gene expression.

According to the teaching, the term "intracellular IFN signalling" relates to the intracellular signaling events and effector functions, in particular antiviral functions, activated by IFNs interacting with their specific receptors and includes the functions of proteins that are induced by IFN, in particular antivirally active effector proteins. In particular, the term "intracellular IFN signalling" includes the signal propagation through and effector functions, in particular antiviral functions, exerted by proteins which are part of the PKR-dependent pathway, in particular PKR and eIF2-alpha, and/or the OAS-dependent pathway, in particular OAS and RNaseL.

The term "inhibiting intracellular IFN signalling" relates to an inhibition or reduction of intracellular IFN signalling and may be achieved by inhibiting expression, activity or activation of proteins which are involved in intracellular IFN signaling, in particular proteins which are part of the PKR-dependent pathway and/or the OAS-dependent pathway. For example, many viruses have evolved mechanisms for counteracting the PKR and OAS/RNase L pathways. These mechanisms may be used according to the teaching for inhibiting intracellular IFN signaling. Those aspects of the present teaching which involve one or more proteins or peptides in inhibiting intracellular IFN signalling, such as proteins or peptides disclosed herein, e.g. one or more proteins or peptides inhibiting the PKR-dependent pathway and/or the OAS-dependent pathway, may involve the provision of nucleic acids, in particular RNA, encoding these one or more proteins or peptides to cells, e.g. by introducing the nucleic acids into cells.

According to the teaching, the PKR-dependent pathway may be inhibited by an agent inhibiting or reducing the activity or activation of PKR or by an agent dephosphorylating eIF2-alpha or preventing its phosphorylation, thereby terminating the PKR-induced signal. For example, intracellular IFN signalling may be inhibited according to the teaching by utilizing any of the viral defense mechanisms against the PKR signaling cascade. In this respect, the teaching may involve the use of decoy dsRNA (e.g. adenovirus VAI RNA; Epstein-Barr virus EBER; HIV TAR), compounds effecting PKR degradation (e.g. poliovirus 2Apro), compounds inhibiting activation of PKR, e.g. through hiding viral dsRNA (e.g. vaccinia virus E3/E3L; reovirus sigma3; influenza virus NS1, herpes simplex virus type 1 (HSV-1) US11), compounds blocking dimerization (e.g. influenza virus p58IPK; Hepatitis C virus NS5A), pseudosubstrates (e.g. vaccinia virus K3/K3L; HIV Tat) or dephosphorylation of substrate (e.g. herpes simplex virus ICP34.5). Vaccinia virus E3 is a 25 kDa dsRNA-binding protein (encoded by gene E3L) that binds and sequesters dsRNA to prevent the activation of PKR and OAS. E3 can bind directly to PKR and inhibits its activity, resulting in reduced phosphorylation of eIF2-alpha. Vaccinia virus gene K3L encodes a 10.5 kDa homolog of the eIF2-alpha subunit that acts as a non-phosphorylable pseudosubstrate of PKR and competitively inhibits phosphorylation of eIF2-alpha. Vaccinia virus C7/C7L inhibits phosphorylation of eIF2-alpha. The ICP34.5 protein from HSV-1 functions as a regulatory subunit of the cellular PP1 phosphatase, directing it to dephosphorylate eIF2-alpha, thereby terminating the PKR-induced signal. The murine cytomegalovirus (MCMV) proteins m142 and m143 have been characterized as dsRNA binding proteins that inhibit PKR activation, phosphorylation of the translation initiation factor eIF2, and a subsequent protein synthesis shutoff.

A decoy RNA is pseudosubstrate RNA that has similar structure to the RNA substrate of an enzyme, in order to make the enzyme bind to the pseudosubstrate rather than to the real substrate, thus blocking the activity of the enzyme.

According to the present teaching, the term "reducing the activity of RNA-dependent protein kinase (PKR)" relates to measures that result in a lower degree of homodimerization of PKR, in a lower degree of autophosphorylation of PKR and/or in a lower degree of phosphorylation of targets which are kinase substrates of PKR such as eIF2-alpha compared to the normal situation, in particular the normal situation in a cell, wherein the activity of PKR is not reduced/has not been reduced by man. Preferably, said term includes all measures that result in a lower degree of autophosphorylation of PKR and/or in a lower degree of phosphorylation of targets which are kinase substrates of PKR.

In one embodiment, reducing the activity of RNA-dependent protein kinase (PKR) in a cell comprises treating the cell with an inhibitor of expression and/or activity of PKR. According to the teaching, the phrase "inhibit expression and/or activity" includes a complete or essentially complete inhibition of expression and/or activity and a reduction in expression and/or activity.

In one embodiment, said PKR inhibitor is directed at the PKR protein and preferably is specific for PKR. PKR can be inhibited in various ways, e.g. through inhibiting PKR autophosphorylation and/or dimerization, providing a PKR pseudo-activator, or providing a PKR pseudo-substrate. The PKR inhibitor may be an agent which is involved in a viral defense mechanism as discussed above. For example, vaccinia virus E3L encodes a dsRNA binding protein that inhibits PKR in virus-infected cells, presumably by sequestering dsRNA activators. K3, also encoded by vaccinia virus, functions as a pseudosubstrate inhibitor by binding to PKR. Thus, providing vaccinia virus E3L may result in inhibition of PKR. Providing adenovirus VAI RNA, HIV Tat or Epstein-Barr virus EBER1 RNA may result in PKR pseudo-activation. Thus, for example, all viral factors, i.e. virally derived inhibitors, blocking PKR activity such as those described herein may be used for reducing the activity of PKR.

In one embodiment, the PKR inhibitor is a chemical inhibitor. Preferably, the PKR inhibitor is an inhibitor of RNA-induced PKR autophosphorylation. Preferably, the PKR inhibitor is an ATP-binding site directed inhibitor of PKR.

In one embodiment, the PKR inhibitor is 6,8-dihydro-8-(1H-imidazol-5-ylmethylene)-7H-pyrrolo[2,3-g]benzothiazol-7-one. In one embodiment, the PKR inhibitor is 2-aminopurine.

In a further embodiment, the inhibitor of activity of PKR is an antibody that specifically binds to PKR. Binding of the antibody to PKR can interfere with the function of PKR, e.g. by inhibiting binding activity or catalytic activity.

In one embodiment, it is envisioned to reduce the activity of PKR in a cell by treating the cell with one or more virally derived inhibitors such as vaccinia virus E3 and/or K3 as well as treating the cell with one or more chemical PKR inhibitors such as 6,8-dihydro-8-(1H-imidazol-5-ylmethylene)-7H-pyrrolo[2,3-g]benzothiazol-7-one and/or 2-aminopurine.

According to the teaching, the OAS-dependent pathway may be inhibited by an agent inhibiting or reducing the activity or activation of OAS and/or RNaseL. For example, vaccinia virus E3 is a 25 kDa dsRNA-binding protein (encoded by gene E3L) that binds and sequesters dsRNA to prevent the activation of OAS.

According to the present teaching, the terms "reducing the activity of OAS" preferably relates to measures that result in a lower degree of production of 2',5'-oligoadenlylates and thus, activation of RNaseL.

In one embodiment, reducing the activity of OAS and/or RNaseL in a cell comprises treating the cell with an inhibitor of expression and/or activity of OAS and/or RNaseL. According to the teaching, the phrase "inhibit expression and/or activity" includes a complete or essentially complete inhibition of expression and/or activity and a reduction in expression and/or activity.

In one embodiment, inhibition of expression of PKR, OAS or RNaseL, in the following referred to as "target protein", may take place by inhibiting the production of or reducing the level of transcript, i.e. mRNA, coding for the target protein, e.g. by inhibiting transcription or inducing degradation of transcript, and/or by inhibiting the production of the target protein, e.g. by inhibiting translation of transcript coding for the target protein. In one embodiment, said inhibitor is specific for a nucleic acid encoding the target protein. In a particular embodiment, the inhibitor of expression of the target protein is an inhibitory nucleic acid (e.g., antisense molecule, ribozyme, iRNA, siRNA or a DNA encoding the same) selectively hybridizing to and being specific for nucleic acid encoding the target protein, thereby inhibiting (e.g., reducing) transcription and/or translation thereof.

In one embodiment, preventing engagement of IFN receptor by extracellular IFN inhibits autocrine and/or paracrine IFN functions. In one embodiment, preventing engagement of IFN receptor by extracellular IFN comprises providing a binding agent for extracellular IFN such as a viral binding agent for extracellular IFN. In one embodiment, the viral binding agent for extracellular IFN is a viral interferon receptor. In one embodiment, the viral binding agent for extracellular IFN is vaccinia virus B18R. In one embodiment, the viral binding agent for extracellular IFN is provided to the cell in the form of a nucleic acid encoding the binding agent, wherein the nucleic acid is preferably RNA.

In one embodiment, inhibiting intracellular IFN signalling comprises inhibiting one or more IFN-inducible antivirally active effector proteins. In one embodiment, the IFN-inducible antivirally active effector protein is selected from the group consisting of RNA-dependent protein kinase (PKR), 2',5'-oligoadenylate synthetase (OAS) and RNaseL. In one embodiment, inhibiting intracellular IFN signalling comprises inhibiting the PKR-dependent pathway and/or the OAS-dependent pathway. In one embodiment, inhibiting the PKR-dependent pathway comprises inhibiting eIF2-alpha phosphorylation. In one embodiment, inhibiting eIF2-alpha phosphorylation comprises inhibiting PKR and/or providing a pseudosubstrate mimicking eIF2-alpha. In one embodiment, the pseudosubstrate mimicking eIF2-alpha is a viral pseudosubstrate mimicking eIF2-alpha. In one embodiment, the viral pseudosubstrate mimicking eIF2-alpha is vaccinia virus K3. In one embodiment, the viral pseudosubstrate mimicking eIF2-alpha is provided to the cell in the form of a nucleic acid encoding the viral pseudosubstrate, wherein the nucleic acid is preferably RNA. In one embodiment, inhibiting PKR comprises treating the cell with at least one PKR inhibitor. In one embodiment, the PKR inhibitor inhibits RNA-induced PKR autophosphorylation. In one embodiment, the PKR inhibitor is an ATP-binding site directed inhibitor of PKR. In one embodiment, the PKR inhibitor is an imidazolo-oxindole compound. In one embodiment, the PKR inhibitor is 6,8-dihydro-8-(1H-imidazol-5-ylmethylene)-7H-pyrrolo[2,3-g]benzothiazol-7-one and/or 2-aminopurine. In one embodiment, the PKR inhibitor is a viral inhibitor of PKR. In one embodiment, the viral inhibitor of PKR is vaccinia virus E3. In one embodiment, the viral inhibitor of PKR is provided to the cell in the form of a nucleic acid encoding the inhibitor, wherein the nucleic acid is preferably RNA. In one embodiment, inhibiting PKR comprises silencing expression of the PKR gene. In one embodiment, inhibiting the OAS-dependent pathway comprises inhibiting activation of RNaseL. In one embodiment, inhibiting the OAS-dependent pathway comprises inhibiting OAS. In one embodiment, inhibiting OAS comprises treating the cell with at least one OAS inhibitor. In one embodiment, the OAS inhibitor is a viral inhibitor of OAS. In one embodiment, the viral inhibitor of OAS is vaccinia virus E3. In one embodiment, the viral inhibitor of OAS is provided to the cell in the form of a nucleic acid encoding the inhibitor.

In one embodiment, the steps of (i) preventing engagement of IFN receptor by extracellular IFN and/or (ii) inhibiting intracellular IFN signalling comprise treating the cell with (i) vaccinia virus B18R and/or (ii) vaccinia virus E3 or vaccinia virus K3, or both. In one embodiment, the vaccinia virus B18R and/or vaccinia virus E3 and/or vaccinia virus K3 are provided to the cell in the form of nucleic acid encoding the vaccinia virus B18R and/or vaccinia virus E3 and/or vaccinia virus K3, either on the same or on two or more different nucleic acid molecules, wherein the nucleic acid is preferably RNA which preferably is introduced into the cell together with the RNA which is to be expressed in the cell and optionally the RNA encoding a virus-derived factor comprising *Toscana virus* NSs protein or a functional variant of *Toscana virus* NSs protein.

The viral escape protein NSs (N) from *Toscana virus* can substitute EKB (E3, K3, B18R) for successful inhibition of an IFN response. Accordingly, the methods of the teaching, in one embodiment, do not comprise providing to a cell (i) an agent preventing engagement of IFN receptor by extracellular IFN and/or (ii) an agent inhibiting intracellular IFN signalling. Accordingly, the methods of the teaching, in one embodiment, do not comprise providing to a cell vaccinia virus B18R and/or vaccinia virus E3 and/or vaccinia virus K3 and preferably do not comprise providing to a cell any of vaccinia virus B18R, vaccinia virus E3 and vaccinia virus K3.

It is to be understood that according to the teaching instead of the IFN inhibitor mentioned above (including a virus-derived factor comprising *Toscana virus* NSs protein or a functional variant of *Toscana virus* NSs protein), nucleic acids encoding the proteins can be provided. The phrase "provided in the form of a nucleic acid" as used herein is to account for this possibility. For example, cells may be transfected with nucleic acid, in particular RNA, encoding the protein and the nucleic acid may be expressed in the cells so as to produce the protein.

A nucleic acid is according to the teaching preferably deoxyribonucleic acid (DNA) or ribonucleic acid (RNA), more preferably RNA, most preferably *in vitro* transcribed RNA (IVT RNA). Nucleic acids include according to the teaching genomic DNA, cDNA, mRNA, recombinantly produced and chemically synthesized molecules. According to the teaching, a nucleic acid may be present as a single-stranded or double-stranded and linear or covalently circularly closed molecule. A nucleic acid can, according to the teaching, be isolated. The term "isolated nucleic acid" means, according to the teaching, that the nucleic acid (i) was amplified *in vitro,* for example via polymerase chain reaction (PCR), (ii) was produced recombinantly by cloning, (iii) was purified, for example, by cleavage and separation by gel electrophoresis, or (iv) was synthesized, for example, by chemical synthesis. A nucleic can be employed for introduction into, i.e. transfection of, cells, in particular, in the form of RNA which can be prepared by *in vitro* transcription from a DNA template. The RNA can moreover be modified before application by stabilizing sequences, capping, and polyadenylation.

As a nucleic acid, in particular RNA, for expression of more than one peptide or protein, either of a nucleic acid type in which the different peptides or proteins are encoded by open reading frames (ORFs) present in different nucleic acid molecules or a nucleic acid type in which the different peptides or proteins are encoded by open reading frames (ORFs) present in the same nucleic acid molecule can be used. Accordingly, RNA encoding more than one peptide or protein such as RNA encoding more than one reprogramming factor may relate to a mixture of different RNA molecules comprising different ORFs encoding different peptides or proteins or to a single RNA molecule comprising different ORFs encoding different peptides or proteins.

In the context of the present teaching, the term "RNA" relates to a molecule which comprises ribonucleotide residues and preferably being entirely or substantially composed of ribonucleotide residues. The term "ribonucleotide" relates to a nucleotide with a hydroxyl group at the 2'-position of a β-D-ribofuranosylgroup. The term "RNA" comprises double-stranded RNA, single stranded RNA, isolated RNA such as partially or completely purified RNA, essentially pure RNA, synthetic RNA, and recombinantly generated RNA such as modified RNA which differs from naturally occurring RNA by addition, deletion, substitution and/or alteration of one or more nucleotides. Such alterations can include addition of non-nucleotide material, such as to the end(s) of a RNA or internally, for example at one or more nucleotides of the RNA. Nucleotides in RNA molecules can also comprise non-standard nucleotides, such as non-naturally occurring nucleotides or chemically synthesized nucleotides or deoxynucleotides. These altered RNAs can be referred to as analogs, particularly analogs of naturally-occurring RNAs. According to the teaching, RNA includes mRNA.

The term "mRNA" means "messenger-RNA" and relates to a transcript which is generated by using a DNA template and encodes a peptide or protein. Typically, mRNA comprises a 5'-UTR, a protein coding region, a 3'-UTR, and a poly(A) sequence. mRNA may be generated by *in vitro* transcription from a DNA template. The *in vitro* transcription methodology is known to the skilled person. For example, there is a variety of *in vitro* transcription kits commercially available.

According to the teaching, RNA encoding a peptide or protein (also termed peptide or protein of interest herein) such as RNA encoding one or more reprogramming factors may be mRNA encoding a peptide or protein or self-replicating RNA such as replicon RNA encoding a peptide or protein. Furthermore, RNA encoding a virus-derived factor comprising *Toscana virus* NSs protein or a functional variant of *Toscana virus* NSs protein for provision to cells according to the teaching may be mRNA encoding the virus-derived factor or self-replicating RNA such as replicon RNA encoding the virus derived factor. In one embodiment of the teaching, RNA encoding a peptide or protein of interest which is mRNA encoding a peptide or protein of interest is co-transfected together with RNA encoding the virus-derived factor which is mRNA encoding the virus-derived factor. In one embodiment of the teaching, RNA encoding a peptide or protein of interest which is replicon RNA encoding a peptide or protein of interest is co-transfected together with RNA encoding the virus-derived factor which is mRNA encoding the virus-derived factor. If the replicon RNA is trans-replicon RNA, also RNA, e.g., mRNA, encoding alphavirus non-structural protein (also termed RNA construct for expressing functional alphavirus non-structural protein herein) may be co-transfected. "Co-transfection" refers to the transfection of different nucleic acids either at the same time point or at different time points.

In one embodiment of the present teaching, RNA is self-replicating RNA, such as single stranded self-replicating RNA. In one embodiment, the self-replicating RNA is single stranded RNA of positive sense. In one embodiment, the self-replicating RNA is viral RNA or RNA derived from viral RNA. In one embodiment, the self-replicating RNA is alphaviral genomic RNA or is derived from alphaviral genomic RNA. In one embodiment, the self-replicating RNA is a viral gene expression vector. In one embodiment, the virus is Semliki forest virus. In one embodiment, the self-replicating RNA contains one or more transgenes. In one embodiment, if the RNA is viral RNA or derived from viral RNA, the transgenes may partially or completely replace viral sequences such as viral sequences encoding structural proteins. In one embodiment, the self-replicating RNA is *in vitro* transcribed RNA.

Alphaviruses are typical representatives of positive-stranded RNA viruses. The genome of alphaviruses encodes non-structural proteins (involved in transcription, modification and replication of viral RNA and in protein modification) and structural proteins (forming the virus particle). There are typically two open reading frames (ORFs) in the genome. The four non-structural proteins (nsP1-nsP4) are typically encoded together by a first ORF beginning near the 5' terminus of the genome, while alphavirus structural proteins are encoded together by a second ORF which is found downstream of the first ORF and extends near the 3' terminus of the genome. Typically, the first ORF is larger than the second ORF, the ratio being roughly 2:1. In cells infected by an alphavirus, only the nucleic acid sequence encoding non-structural proteins is translated from the genomic RNA, while the genetic information encoding structural proteins is translatable from a subgenomic transcript, which is an RNA molecule that resembles eukaryotic messenger RNA (mRNA; Gould et al., 2010, Antiviral Res., vol. 87 pp. 111-124). Following infection, i.e. at early stages of the viral life cycle, the (+) stranded genomic RNA directly acts like a messenger RNA for the translation of the open reading frame encoding the non-structural poly-protein (nsP1234). In some alphaviruses, there is an opal stop codon between the coding sequences of nsP3 and nsP4: polyprotein P123, containing nsP1, nsP2, and nsP3, is produced when translation terminates at the opal stop codon, and polyprotein P1234, containing in addition nsP4, is produced upon readthrough of this opal codon (Strauss & Strauss, Microbiol. Rev., 1994, vol. 58, pp. 491-562; Rupp et al., 2015, J. Gen. Virology, vol. 96, pp. 2483-2500). nsP1234 is autoproteolytically cleaved into the fragments nsP123 and nsP4. The polypeptides nsP123 and nsP4 associate to form the (-) strand replicase complex that transcribes (-) stranded RNA, using the (+) stranded genomic RNA as template. Typically at later stages, the nsP123 fragment is completely cleaved into individual proteins nsP1, nsP2 and nsP3 (Shirako & Strauss, 1994, J. Virol., vol. 68, pp. 1874-1885). All four proteins form the (+) strand replicase complex that synthesizes new (+) stranded genomes, using the (-) stranded complement of genomic RNA as template (Kim et al., 2004, Virology, vol. 323, pp. 153-163, Vasiljeva et al., 2003, J. Biol. Chem. vol. 278, pp. 41636-41645).

Alphavirus structural proteins (core nucleocapsid protein C, envelope protein E2 and envelope protein E1, all constituents of the virus particle) are typically encoded by one single open reading frame under control of a subgenomic promoter (Strauss & Strauss, Microbiol. Rev., 1994, vol. 58, pp. 491-562). The subgenomic promoter is recognized by alphaviral non-structural proteins acting in *cis.* In particular, alphavirus replicase synthesizes a (+) stranded subgenomic transcript using the (-) stranded complement of genomic RNA as template. The (+) stranded subgenomic transcript encodes the alphavirus structural proteins (Kim et al., 2004, Virology, vol. 323, pp. 153-163, Vasiljeva et al., 2003, J. Biol. Chem. vol. 278, pp. 41636-41645). The subgenomic RNA transcript serves as template for translation of the open reading frame encoding the structural proteins as one poly-protein, and the poly-protein is cleaved to yield the structural proteins. At a late stage of alphavirus infection in a host cell, a packaging signal which is located within the coding sequence of nsP2 ensures selective packaging of genomic RNA into budding virions, packaged by structural proteins (White et al., 1998, J. Virol., vol. 72, pp. 4320-4326).

In embodiments of the teaching, the self-replicating RNA may be an RNA replicon which is a cis-replicon or trans-replicon. For example, the open reading frame encoding alphaviral structural proteins may be replaced by an open reading frame encoding a protein of interest. Alphavirus-based trans-replication systems rely on alphavirus nucleotide sequence elements on two separate nucleic acid molecules: one nucleic acid molecule encodes a viral replicase (typically as poly-protein nsP1234), and the other nucleic acid molecule is capable of being replicated by said replicase *in trans* (hence the designation trans-replication system). *trans*-replication requires the presence of both these nucleic acid molecules in a given host cell. The nucleic acid molecule capable of being replicated by the replicase *in trans* must comprise certain alphaviral sequence elements to allow recognition and RNA synthesis by the alphaviral replicase.

According to the present teaching, "RNA replication" generally refers to an RNA molecule synthesized based on the nucleotide sequence of a given RNA molecule (template RNA molecule). The RNA molecule that is synthesized may be e.g. identical or complementary to the template RNA molecule. In general, RNA replication may occur via synthesis of a DNA intermediate, or may occur directly by RNA-dependent RNA replication mediated by a RNA-dependent RNA polymerase (RdRP). In the case of alphaviruses, RNA replication does not occur via a DNA intermediate, but is mediated by a RNA-dependent RNA polymerase (RdRP): a template RNA strand (first RNA strand) - or a part thereof - serves as template for the synthesis of a second RNA strand that is complementary to the first RNA strand or to a part thereof. The second RNA strand - or a part thereof - may in turn optionally serve as a template for synthesis of a third RNA strand that is complementary to the second RNA strand or to a part thereof. Thereby, the third RNA strand is identical to the first RNA strand or to a part thereof. Thus, RNA-dependent RNA polymerase is capable of directly synthesizing a complementary RNA strand of a template, and of indirectly synthesizing an identical RNA strand (via a complementary intermediate strand).

A nucleic acid construct that is capable of being replicated by a replicase, preferably an alphaviral replicase, is termed replicon. According to the teaching, the term "replicon" defines a RNA molecule that can be replicated by RNA-dependent RNA polymerase, yielding - without DNA intermediate - one or multiple identical or essentially identical copies of the RNA replicon. "Without DNA intermediate" means that no deoxyribonucleic acid (DNA) copy or complement of the replicon is formed in the process of forming the copies of the RNA replicon, and/or that no deoxyribonucleic acid (DNA) molecule is used as a template in the process of forming the copies of the RNA replicon, or complement thereof. The replicase function is typically provided by functional alphavirus non-structural protein.

The RNA replicon of the teaching preferably comprises a 5' replication recognition sequence and a 3' replication recognition sequence. A replication recognition sequence is a nucleic acid sequence that can be recognized by functional alphavirus non-structural protein.

In one embodiment, the 5' replication recognition sequence and the 3' replication recognition sequence are capable of directing replication of the RNA replicon according to the present teaching in the presence of functional alphavirus non-structural protein. Thus, when present alone or preferably together, these recognition sequences direct replication of the RNA replicon in the presence of functional alphavirus non-structural protein.

It is preferable that a functional alphavirus non-structural protein is provided in cis (encoded as protein of interest by an open reading frame on the replicon) or in trans (encoded as protein of interest by an open reading frame on a separate replicase construct), that is capable of recognizing both the 5' replication recognition sequence and the 3' replication recognition sequence of the replicon.

The RNA replicon according to the present teaching is preferably a single stranded RNA molecule. The RNA replicon according to the present teaching is typically a (+) stranded RNA molecule. In one embodiment, the RNA replicon of the present teaching is an isolated nucleic acid molecule.

According to the teaching, a RNA replicon may be derived from an alphavirus selected from the group consisting of the following: Barmah Forest virus complex (comprising Barmah Forest virus); Eastern equine encephalitis complex (comprising seven antigenic types of Eastern equine encephalitis virus); Middelburg virus complex (comprising Middelburg virus); Ndumu virus complex (comprising Ndumu virus); Semliki Forest virus complex (comprising Bebaru virus, Chikungunya virus, Mayaro virus and its subtype Una virus, O'Nyong Nyong virus, and its subtype Igbo-Ora virus, Ross River virus and its subtypes Bebaru virus, Getah virus, Sagiyama virus, Semliki Forest virus and its subtype Me Tri virus); Venezuelan equine encephalitis complex (comprising Cabassou virus, Everglades virus, Mosso das Pedras virus, Mucambo virus, Paramana virus, Pixuna virus, Rio Negro virus, Trocara virus and its subtype Bijou Bridge virus, Venezuelan equine encephalitis virus); Western equine encephalitis complex (comprising Aura virus, Babanki virus, Kyzylagach virus, Sindbis virus, Ockelbo virus, Whataroa virus, Buggy Creek virus, Fort Morgan virus, Highlands J virus, Western equine encephalitis virus); and some unclassified viruses including Salmon pancreatic disease virus; Sleeping Disease virus; Southern elephant seal virus; Tonate virus. More preferably, the alphavirus is selected from the group consisting of Semliki Forest virus complex (comprising the virus types as indicated above, including Semliki Forest virus), Western equine encephalitis complex (comprising the virus types as indicated above, including Sindbis virus), Eastern equine encephalitis virus (comprising the virus types as indicated above), Venezuelan equine encephalitis complex (comprising the virus types as indicated above, including Venezuelan equine encephalitis virus). In a further preferred embodiment, the alphavirus is Semliki Forest virus. In an alternative further preferred embodiment, the alphavirus is Sindbis virus. In an alternative further preferred embodiment, the alphavirus is Venezuelan equine encephalitis virus.

RNA molecules according to the teaching may optionally be characterized by further features, e.g. by a 5'-cap, a 5'-UTR, a 3'-UTR, a poly(A) sequence, and/or adaptation of the codon usage. Details are described in the following.

In some embodiments, the RNA (mRNA and/or replicon RNA) according to the present teaching comprises a 5'-cap.

The terms "5'-cap", "cap", "5'-cap structure", "cap structure" are used synonymously to refer to a dinucleotide that is found on the 5' end of some eukaryotic primary transcripts such as precursor messenger RNA. A 5'-cap is a structure wherein a (optionally modified) guanosine is bonded to the first nucleotide of an mRNA molecule via a 5' to 5' triphosphate linkage (or modified triphosphate linkage in the case of certain cap analogs). The terms can refer to a conventional cap or to a cap analog.

"RNA which comprises a 5'-cap" or "RNA which is provided with a 5'-cap" or "RNA which is modified with a 5'-cap" or "capped RNA" refers to RNA which comprises a 5'-cap. For example, providing an RNA with a 5'-cap may be achieved by *in vitro* transcription of a DNA template in presence of said 5'-cap, wherein said 5'-cap is co-transcriptionally incorporated into the generated RNA strand, or the RNA may be generated, for example, by *in vitro* transcription, and the 5'-cap may be attached to the RNA post-transcriptionally using capping enzymes, for example, capping enzymes of vaccinia virus. In capped RNA, the 3' position of the first base of a (capped) RNA molecule is linked to the 5' position of the subsequent base of the RNA molecule ("second base") via a phosphodiester bond.

Presence of a cap on an RNA molecule is strongly preferred if translation of a nucleic acid sequence encoding a protein at early stages after introduction of the respective RNA into host cells or into a host organism is desired. For example, presence of a cap allows that a gene of interest encoded by RNA is translated efficiently at early stages after introduction of the respective RNA into host cells. "Early stages" typically means within the first 1 hour, or within the first two hours, or within the first three hours after introduction of the RNA.

Presence of a cap on an RNA replicon is also preferred if it is desired that translation occurs in the absence of functional replicase, or when only minor levels of replicase are present in a host cell. For example, even if a nucleic acid molecule encoding replicase is introduced into a host cell, at early stages after introduction the levels of replicase will typically be minor. According to the teaching, it is preferred that the RNA construct for expressing functional alphavirus non-structural protein comprises a 5'-cap.

In particular when the RNA replicon according to the present teaching is not used or provided together with a second nucleic acid molecule (e.g. mRNA) that encodes functional alphavirus non-structural protein, it is preferred that the RNA replicon comprises a 5'-cap. Independently, the RNA replicon may also comprise a 5'-cap even when it is used or provided together with a second nucleic acid molecule that encodes functional alphavirus non-structural protein.

The term "conventional 5'-cap" refers to a naturally occurring 5'-cap, preferably to the 7-methylguanosine cap. In the 7-methylguanosine cap, the guanosine of the cap is a modified guanosine wherein the modification consists of a methylation at the 7-position.

In the context of the present teaching, the term "5'-cap analog" refers to a molecular structure that resembles a conventional 5'-cap, but is modified to possess the ability to stabilize RNA if attached thereto, preferably *in vivo* and/or in a cell. A cap analog is not a conventional 5'-cap.

For the case of eukaryotic mRNA, the 5'-cap has been generally described to be involved in efficient translation of mRNA: in general, in eukaryotes, translation is initiated only at the 5' end of a messenger RNA (mRNA) molecule, unless an internal ribosomal entry site (IRES) is present. Eukaryotic cells are capable of providing an RNA with a 5'-cap during transcription in the nucleus: newly synthesized mRNAs are usually modified with a 5'-cap structure, e.g. when the transcript reaches a length of 20 to 30 nucleotides. First, the 5' terminal nucleotide pppN (ppp representing triphosphate; N representing any nucleoside) is converted in the cell to 5' GpppN by a capping enzyme having RNA 5'-triphosphatase and guanylyltransferase activities. The GpppN may subsequently be methylated in the cell by a second enzyme with (guanine-7)-methyltransferase activity to form the mono-methylated m⁷GpppN cap. In one embodiment, the 5'-cap used in the present teaching is a natural 5'-cap.

In the present teaching, a natural 5'-cap dinucleotide is typically selected from the group consisting of a non-methylated cap dinucleotide (G(5')ppp(5')N; also termed GpppN) and a methylated cap dinucleotide ((m⁷G(5')ppp(5')N; also termed m⁷GpppN). m⁷GpppN (wherein N is G) is represented by the following formula:

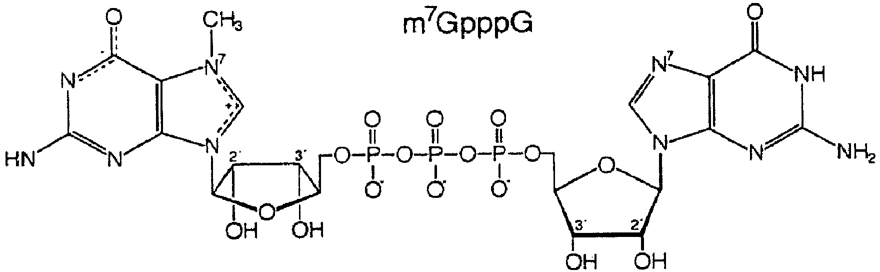

Capped RNA of the present teaching can be prepared *in vitro,* and therefore, does not depend on a capping machinery in a host cell. The most frequently used method to make capped RNAs *in vitro* is to transcribe a DNA template with either a bacterial or bacteriophage RNA polymerase in the presence of all four ribonucleoside triphosphates and a cap dinucleotide such as m⁷G(5')ppp(5')G (also called m⁷GpppG). The RNA polymerase initiates transcription with a nucleophilic attack by the 3'-OH of the guanosine moiety of m⁷GpppG on the α-phosphate of the next templated nucleoside triphosphate (pppN), resulting in the intermediate m⁷GpppGpN (wherein N is the second base of the RNA molecule). The formation of the competing GTP-initiated product pppGpN is suppressed by setting the molar ratio of cap to GTP between 5 and 10 during *in vitro* transcription.

In preferred embodiments of the present teaching, the 5'-cap (if present) is a 5'-cap analog. These embodiments are particularly suitable if the RNA is obtained by *in vitro* transcription, e.g. is an *in vitro* transcribed RNA (IVT-RNA). Cap analogs have been initially described to facilitate large scale synthesis of RNA transcripts by means of *in vitro* transcription.

For messenger RNA, some cap analogs (synthetic caps) have been generally described to date, and they can all be used in the context of the present teaching. Ideally, a cap analog is selected that is associated with higher translation efficiency and/or increased resistance to *in vivo* degradation and/or increased resistance to *in vitro* degradation.

Preferably, a cap analog is used that can only be incorporated into an RNA chain in one orientation. Pasquinelli et al. (1995, RNA J., vol., 1, pp. 957-967) demonstrated that during *in vitro* transcription, bacteriophage RNA polymerases use the 7-methylguanosine unit for initiation of transcription, whereby around 40-50% of the transcripts with cap possess the cap dinucleotide in a reverse orientation (i.e., the initial reaction product is Gpppm⁷GpN). Compared to the RNAs with a correct cap, RNAs with a reverse cap are not functional with respect to translation of a nucleic acid sequence into protein. Thus, it is desirable to incorporate the cap in the correct orientation, i.e., resulting in an RNA with a structure essentially corresponding to m⁷GpppGpN etc. It has been shown that the reverse integration of the cap-dinucleotide is inhibited by the substitution of either the 2'- or the 3'-OH group of the methylated guanosine unit (Stepinski et al., 2001; RNA J., vol. 7, pp. 1486-1495; Peng et al., 2002; Org. Lett., vol. 24, pp. 161-164). RNAs which are synthesized in presence of such "anti reverse cap analogs" are translated more efficiently than RNAs which are *in vitro* transcribed in presence of the conventional 5'-cap m⁷GpppG. To that end, one cap analog in which the 3' OH group of the methylated guanosine unit is replaced by OCH₃ is described e.g. by Holtkamp et al., 2006, Blood, vol. 108, pp. 4009-4017 (7-methyl(3'-O-methyl)GpppG; anti-reverse cap analog (ARCA)). ARCA is a suitable cap dinucleotide according to the present teaching.

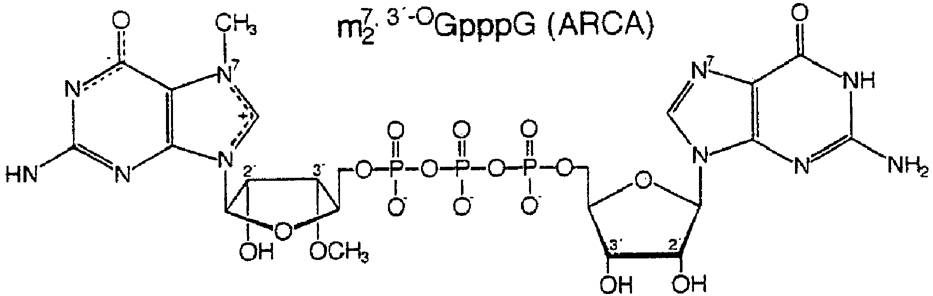

In a preferred embodiment of the present teaching, the RNA of the present teaching is essentially not susceptible to decapping. This is important because, in general, the amount of protein produced from synthetic mRNAs introduced into cultured mammalian cells is limited by the natural degradation of mRNA. One *in vivo* pathway for mRNA degradation begins with the removal of the mRNA cap. This removal is catalyzed by a heterodimeric pyrophosphatase, which contains a regulatory subunit (Dcp1) and a catalytic subunit (Dcp2). The catalytic subunit cleaves between the α and β phosphate groups of the triphosphate bridge. In the present teaching, a cap analog may be selected or present that is not susceptible, or less susceptible, to that type of cleavage. A suitable cap analog for this purpose may be selected from a cap dinucleotide according to formula (I): 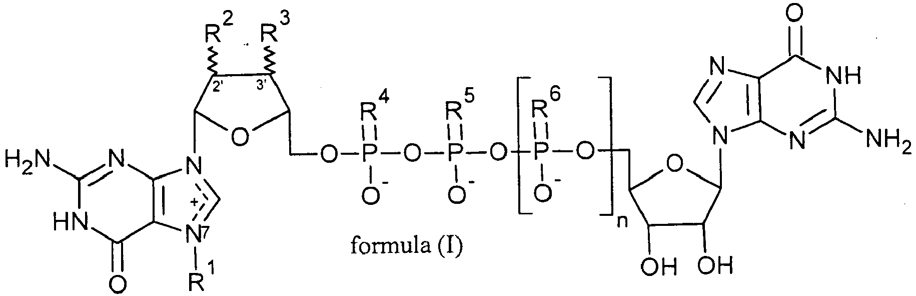
wherein R¹ is selected from the group consisting of optionally substituted alkyl, optionally substituted alkenyl, optionally substituted alkynyl, optionally substituted cycloalkyl, optionally substituted heterocyclyl, optionally substituted aryl, and optionally substituted heteroaryl,
R² and R³ are independently selected from the group consisting of H, halo, OH, and optionally substituted alkoxy, or R² and R³ together form O-X-O, wherein X is selected from the group consisting of optionally substituted CH₂, CH₂CH₂, CH₂CH₂CH₂, CH₂CH(CH₃), and C(CH₃)₂, or R² is combined with the hydrogen atom at position 4' of the ring to which R² is attached to form -O-CH₂- or -CH₂-O-,
R⁵ is selected from the group consisting of S, Se, and BHs,
R⁴ and R⁶ are independently selected from the group consisting of O, S, Se, and BH₃.
n is 1, 2, or 3.

Preferred embodiments for R¹, R², R3, R⁴, R⁵, R⁶ are disclosed in WO 2011/015347 A1 and may be selected accordingly in the present teaching.

For example, in a preferred embodiment of the present teaching, the RNA of the present teaching comprises a phosphorothioate-cap-analog. Phosphorothioate-cap-analogs are specific cap analogs in which one of the three non-bridging O atoms in the triphosphate chain is replaced with an S atom, i.e. one of R⁴, R⁵ or R⁶ in Formula (I) is S. Phosphorothioate-cap-analogs have been described by J. Kowalska et al., 2008, RNA, vol. 14, pp. 1119-1131, as a solution to the undesired decapping process, and thus to increase the stability of RNA *in vivo.* In particular, the substitution of an oxygen atom for a sulphur atom at the beta-phosphate group of the 5'-cap results in stabilization against Dcp2. In that embodiment, which is preferred in the present teaching, R⁵ in Formula (I) is S; and R⁴ and R⁶ are O.

In a further preferred embodiment of the present teaching, the RNA of the present teaching comprises a phosphorothioate-cap-analog wherein the phosphorothioate modification of the RNA 5'-cap is combined with an "anti-reverse cap analog" (ARCA) modification. Respective ARCA-phosphorothioate-cap-analogs are described in WO 2008/157688 A2, and they can all be used in the RNA of the present teaching. In that embodiment, at least one of R² or R³ in Formula (I) is not OH, preferably one among R² and R³ is methoxy (OCH₃), and the other one among R² and R³ is preferably OH. In a preferred embodiment, an oxygen atom is substituted for a sulphur atom at the beta-phosphate group (so that R⁵ in Formula (I) is S; and R⁴ and R⁶ are O). It is believed that the phosphorothioate modification of the ARCA ensures that the α, β, and γ phosphorothioate groups are precisely positioned within the active sites of cap-binding proteins in both the translational and decapping machinery. At least some of these analogs are essentially resistant to pyrophosphatase Dcp1/Dcp2. Phosphorothioate-modified ARCAs were described to have a much higher affinity for eIF4E than the corresponding ARCAs lacking a phosphorothioate group.

A respective cap analog that is particularly preferred in the present teaching, i.e., m_{2'}^{7,2'-O}GppₛpG, is termed beta-S-ARCA (WO 2008/157688 A2; Kuhn et al., Gene Ther., 2010, vol. 17, pp. 961-971). Thus, in one embodiment of the present teaching, the RNA of the present teaching is modified with beta-S-ARCA. beta-S-ARCA is represented by the following structure:

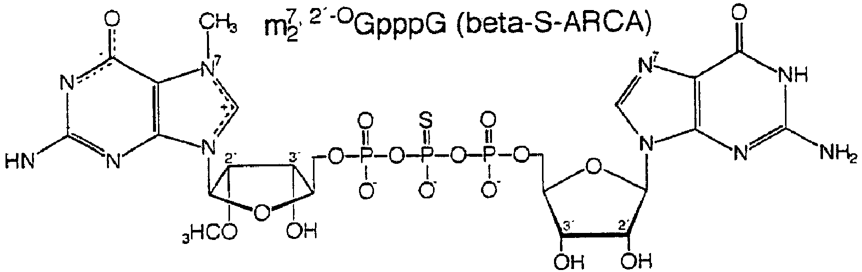

In general, the replacement of an oxygen atom for a sulphur atom at a bridging phosphate results in phosphorothioate diastereomers which are designated D1 and D2, based on their elution pattern in HPLC. Briefly, the D1 diastereomer of beta-S-ARCA" or "beta-S-ARCA(D1)" is the diastereomer of beta-S-ARCA which elutes first on an HPLC column compared to the D2 diastereomer of beta-S-ARCA (beta-S-ARCA(D2)) and thus exhibits a shorter retention time. Determination of the stereochemical configuration by HPLC is described in WO 2011/015347 A1.

In a first particularly preferred embodiment of the present teaching, RNA of the present teaching is modified with the beta-S-ARCA(D2) diastereomer. The two diastereomers of beta-S-ARCA differ in sensitivity against nucleases. It has been shown that RNA carrying the D2 diastereomer of beta-S-ARCA is almost fully resistant against Dcp2 cleavage (only 6% cleavage compared to RNA which has been synthesized in presence of the unmodified ARCA 5'-cap), whereas RNA with the beta-S-ARCA(D1) 5'-cap exhibits an intermediary sensitivity to Dcp2 cleavage (71% cleavage). It has further been shown that the increased stability against Dcp2 cleavage correlates with increased protein expression in mammalian cells. In particular, it has been shown that RNAs carrying the beta-S-ARCA(D2) cap are more efficiently translated in mammalian cells than RNAs carrying the beta-S-ARCA(D1) cap. Therefore, in one embodiment of the present teaching, RNA of the present teaching is modified with a cap analog according to Formula (I), characterized by a stereochemical configuration at the P atom comprising the substituent R⁵ in Formula (I) that corresponds to that at the P_{β} atom of the D2 diastereomer of beta-S-ARCA. In that embodiment, R⁵ in Formula (I) is S; and R⁴ and R⁶ are O. Additionally, at least one of R² or R³ in Formula (I) is preferably not OH, preferably one among R² and R³ is methoxy (OCH3), and the other one among R² and R³ is preferably OH.

In a second particularly preferred embodiment, RNA of the present teaching is modified with the beta-S-ARCA(D1) diastereomer. This embodiment is particularly suitable for transfer of capped RNA into immature antigen presenting cells, such as for vaccination purposes. It has been demonstrated that the beta-S-ARCA(D1) diastereomer, upon transfer of respectively capped RNA into immature antigen presenting cells, is particularly suitable for increasing the stability of the RNA, increasing translation efficiency of the RNA, prolonging translation of the RNA, increasing total protein expression of the RNA, and/or increasing the immune response against an antigen or antigen peptide encoded by said RNA (Kuhn et al., 2010, Gene Ther., vol. 17, pp. 961-971). Therefore, in an alternative embodiment of the present teaching, RNA of the present teaching is modified with a cap analog according to Formula (I), characterized by a stereochemical configuration at the P atom comprising the substituent R⁵ in Formula (I) that corresponds to that at the P_{β} atom of the D1 diastereomer of beta-S-ARCA. Respective cap analogs and embodiments thereof are described in WO 2011/015347 A1 and Kuhn et al., 2010, Gene Ther., vol. 17, pp. 961-971. Any cap analog described in WO 2011/015347 A1, wherein the stereochemical configuration at the P atom comprising the substituent R⁵ corresponds to that at the P_{β} atom of the D1 diastereomer of beta-S-ARCA, may be used in the present teaching. Preferably, R⁵ in Formula (I) is S; and R⁴ and R⁶ are O. Additionally, at least one of R² or R³ in Formula (I) is preferably not OH, preferably one among R² and R³ is methoxy (OCH3), and the other one among R² and R³ is preferably OH.

In one embodiment, RNA of the present teaching is modified with a 5'-cap structure according to Formula (I) wherein any one phosphate group is replaced by a boranophosphate group or a phosphoroselenoate group. Such caps have increased stability both *in vitro* and *in vivo.* Optionally, the respective compound has a 2'-O- or 3'-O-alkyl group (wherein alkyl is preferably methyl); respective cap analogs are termed BH₃-ARCAs or Se-ARCAs. Compounds that are particularly suitable for capping of mRNA include the β-BH₃-ARCAs and β-Se-ARCAs, as described in WO 2009/149253 A2. For these compounds, a stereochemical configuration at the P atom comprising the substituent R⁵ in Formula (I) that corresponds to that at the P_{β} atom of the D1 diastereomer of beta-S-ARCA is preferred.

The term "untranslated region" or "UTR" relates to a region in a DNA molecule which is transcribed but is not translated into an amino acid sequence, or to the corresponding region in an RNA molecule, such as an mRNA molecule. An untranslated region (UTR) can be present 5' (upstream) of an open reading frame (5'-UTR) and/or 3' (downstream) of an open reading frame (3'-UTR).

A 3'-UTR, if present, is located at the 3' end of a gene, downstream of the termination codon of a protein-encoding region, but the term "3'-UTR" does preferably not include the poly(A) tail. Thus, the 3'-UTR is upstream of the poly(A) tail (if present), e.g. directly adjacent to the poly(A) tail.

A 5'-UTR, if present, is located at the 5' end of a gene, upstream of the start codon of a protein-encoding region. A 5'-UTR is downstream of the 5'-cap (if present), e.g. directly adjacent to the 5'-cap.

5'- and/or 3'-untranslated regions may, according to the teaching, be functionally linked to an open reading frame, so as for these regions to be associated with the open reading frame in such a way that the stability and/or translation efficiency of the RNA comprising said open reading frame are increased.

In some embodiments, mRNA according to the present teaching comprises a 5'-UTR and/or a 3'-UTR.

In a preferred embodiment, mRNA according to the present teaching comprises
(1) a 5'-UTR,
(2) an open reading frame, and
(3) a 3'-UTR.

UTRs are implicated in stability and translation efficiency of RNA. Both can be improved, besides structural modifications concerning the 5'-cap and/or the 3' poly(A)-tail as described herein, by selecting specific 5' and/or 3' untranslated regions (UTRs). Sequence elements within the UTRs are generally understood to influence translational efficiency (mainly 5'-UTR) and RNA stability (mainly 3'-UTR). It is preferable that a 5'-UTR is present that is active in order to increase the translation efficiency and/or stability of the RNA. Independently or additionally, it is preferable that a 3'-UTR is present that is active in order to increase the translation efficiency and/or stability of the RNA.

The terms "active in order to increase the translation efficiency" and/or "active in order to increase the stability", with reference to a first nucleic acid sequence (e.g. a UTR), means that the first nucleic acid sequence is capable of modifying, in a common transcript with a second nucleic acid sequence, the translation efficiency and/or stability of said second nucleic acid sequence in such a way that said translation efficiency and/or stability is increased in comparison with the translation efficiency and/or stability of said second nucleic acid sequence in the absence of said first nucleic acid sequence.

A 3'-UTR typically has a length of 200 to 2000 nucleotides, e.g. 500 to 1500 nucleotides. The 3'-untranslated regions of immunoglobulin mRNAs are relatively short (fewer than about 300 nucleotides), while the 3'-untranslated regions of other genes are relatively long. For example, the 3'-untranslated region of tPA is about 800 nucleotides in length, that of factor VIII is about 1800 nucleotides in length and that of erythropoietin is about 560 nucleotides in length. The 3'-untranslated regions of mammalian mRNA typically have a homology region known as the AAUAAA hexanucleotide sequence. This sequence is presumably the poly(A) attachment signal and is frequently located from 10 to 30 bases upstream of the poly(A) attachment site. 3'-untranslated regions may contain one or more inverted repeats which can fold to give stem-loop structures which act as barriers for exoribonucleases or interact with proteins known to increase RNA stability (e.g. RNA-binding proteins).

In some embodiments, the RNA according to the present teaching comprises a 3'-poly(A) sequence.

According to the teaching, in one embodiment, a poly(A) sequence comprises or essentially consists of or consists of at least 20, preferably at least 26, preferably at least 40, preferably at least 80, preferably at least 100 and preferably up to 500, preferably up to 400, preferably up to 300, preferably up to 200, and in particular up to 150, A nucleotides, and in particular about 120 A nucleotides. In this context "essentially consists of" means that most nucleotides in the poly(A) sequence, typically at least 50 %, and preferably at least 75 % by number of nucleotides in the "poly(A) sequence", are A nucleotides (adenylate), but permits that remaining nucleotides are nucleotides other than A nucleotides, such as U nucleotides (uridylate), G nucleotides (guanylate), C nucleotides (cytidylate). In this context "consists of" means that all nucleotides in the poly(A) sequence, i.e. 100 % by number of nucleotides in the poly(A) sequence, are A nucleotides. The term "A nucleotide" or "A" refers to adenylate.

Indeed, it has been demonstrated that a 3' poly(A) sequence of about 120 A nucleotides has a beneficial influence on the levels of RNA in transfected eukaryotic cells, as well as on the levels of protein that is translated from an open reading frame that is present upstream (5') of the 3' poly(A) sequence (Holtkamp et al., 2006, Blood, vol. 108, pp. 4009-4017).

The present teaching provides for a 3' poly(A) sequence to be attached during RNA transcription, i.e. during preparation of *in vitro* transcribed RNA, based on a DNA template comprising repeated dT nucleotides (deoxythymidylate) in the strand complementary to the coding strand. The DNA sequence encoding a poly(A) sequence (coding strand) is referred to as poly(A) cassette.

In a preferred embodiment of the present teaching, the 3' poly(A) cassette present in the coding strand of DNA essentially consists of dA nucleotides, but is interrupted by a random sequence having an equal distribution of the four nucleotides (dA, dC, dG, dT). Such random sequence may be 5 to 50, preferably 10 to 30, more preferably 10 to 20 nucleotides in length. Such a cassette is disclosed in WO 2016/005004 A1. Any poly(A) cassette disclosed in WO 2016/005004 A1 may be used in the present teaching. A poly(A) cassette that essentially consists of dA nucleotides, but is interrupted by a random sequence having an equal distribution of the four nucleotides (dA, dC, dG, dT) and having a length of e.g. 5 to 50 nucleotides shows, on DNA level, constant propagation of plasmid DNA in *E.coli* and is still associated, on RNA level, with the beneficial properties with respect to supporting RNA stability and translational efficiency.

Consequently, in a preferred embodiment of the present teaching, the 3' poly(A) sequence contained in an RNA molecule described herein essentially consists of A nucleotides, but is interrupted by a random sequence having an equal distribution of the four nucleotides (A, C, G, U). Such random sequence may be 5 to 50, preferably 10 to 30, more preferably 10 to 20 nucleotides in length.

The nucleic acid sequences specified herein, in particular transcribable and coding nucleic acid sequences, may be functionally linked with any expression control sequences, which may be homologous or heterologous to said nucleic acid sequences, with the term "homologous" referring to the fact that a nucleic acid sequence is also functionally linked naturally to the expression control sequence, and the term "heterologous" referring to the fact that a nucleic acid sequence is not naturally functionally linked to the expression control sequence.

A transcribable nucleic acid sequence or a nucleic acid sequence coding for a peptide or protein, and an expression control sequence are "functionally" linked to one another, if they are covalently linked to one another in such a way that transcription or expression of the transcribable or coding nucleic acid sequence is under the control or under the influence of the expression control sequence. If the nucleic acid sequence is to be translated into a functional peptide or protein, induction of an expression control sequence functionally linked to the coding sequence results in transcription of said coding sequence, without causing a frame shift in the coding sequence or the coding sequence being unable to be translated into the desired peptide or protein.

The term "expression control sequence" comprises according to the teaching promoters, ribosome-binding sequences and other control elements which control transcription of a gene or translation of RNA. In particular embodiments of the teaching, the expression control sequences can be regulated. The precise structure of expression control sequences may vary depending on the species or cell type but usually includes 5'-untranscribed and 5'- and 3'-untranslated sequences involved in initiating transcription and translation, respectively, such as TATA box, capping sequence, CAAT sequence and the like. More specifically, 5'-untranscribed expression control sequences include a promoter region which encompasses a promoter sequence for transcription control of the functionally linked gene. Expression control sequences may also include enhancer sequences or upstream activator sequences.

The term "promoter" or "promoter region" refers to a DNA sequence upstream (5') of the coding sequence of a gene, which controls expression of said coding sequence by providing a recognition and binding site for RNA polymerase. The promoter region may include further recognition or binding sites for further factors involved in regulating transcription of said gene. A promoter may control transcription of a prokaryotic or eukaryotic gene. A promoter may be "inducible" and initiate transcription in response to an inducer, or may be "constitutive" if transcription is not controlled by an inducer. An inducible promoter is expressed only to a very small extent or not at all, if an inducer is absent. In the presence of the inducer, the gene is "switched on" or the level of transcription is increased. This is usually mediated by binding of a specific transcription factor.

Examples of promoters preferred according to the teaching are promoters for SP6, T3 or T7 polymerase.

According to the teaching, the term "expression" is used in its most general meaning and comprises expression of RNA and/or protein. It also comprises partial expression of nucleic acids. Furthermore, expression may be transient or stable. With respect to RNA, the term "expression" or "translation" relates to the process in the ribosomes of a cell by which a strand of messenger RNA directs the assembly of a sequence of amino acids to make a peptide or protein.

In the context of the present teaching, the term "transcription" relates to a process, wherein the genetic code in a DNA sequence is transcribed into RNA. Subsequently, the RNA may be translated into protein. According to the present teaching, the term "transcription" comprises "*in vitro* transcription", wherein the term "*in vitro* transcription" relates to a process wherein RNA, in particular mRNA, is *in vitro* synthesized in a cell-free system. Preferably, cloning vectors are applied for the generation of transcripts. These cloning vectors are generally designated as transcription vectors and are according to the present teaching encompassed by the term "vector". According to the present teaching, RNA preferably is *in vitro* transcribed RNA (IVT-RNA) and may be obtained by *in vitro* transcription of an appropriate DNA template. The promoter for controlling transcription can be any promoter for any RNA polymerase. A DNA template for *in vitro* transcription may be obtained by cloning of a nucleic acid, in particular cDNA, and introducing it into an appropriate vector for *in vitro* transcription. The cDNA may be obtained by reverse transcription of RNA.

Terms such as "enhancement of expression", "enhanced expression" or "increased expression" mean in the context of the present teaching that the amount of peptide or protein expressed by a given number of RNA molecules is higher than the amount of peptide or protein expressed by the same number of RNA molecules, wherein expression of the RNA molecules is performed under the same conditions except the condition which results in the enhanced or increased expression of the RNA, such as providing a virus-derived factor comprising *Toscana virus* NSs protein or a functional variant of *Toscana virus* NSs protein to the cell versus not providing a virus-derived factor comprising *Toscana virus* NSs protein or a functional variant of *Toscana virus* NSs protein to the cell. In this context, "same conditions" refer to a situation wherein the same RNA sequences encoding the same peptide or protein are introduced by the same means into the same cells, the cells are cultured under the same conditions (except the condition which results in the enhanced or increased expression) and the amount of peptide or protein is measured by the same means. The amount of peptide or protein may be given in moles, or by weight, e.g. in grams, or by mass or by polypeptide activity, e.g. if the peptide or protein is an enzyme it may be given as catalytic activity or if the peptide or protein is an antibody or antigen or a receptor it may be given as binding affinity. In one embodiment, terms such as "enhancement of expression", "enhanced expression" or "increased expression" mean in the context of the present teaching that the amount of peptide or protein expressed by a given number of RNA molecules and within a given period of time is higher than the amount of peptide or protein expressed by the same number of RNA molecules and within the same period of time. For example, the maximum value of peptide or protein expressed by a given number of RNA molecules at a particular time point may be higher than the maximum value of peptide or protein expressed by the same number of RNA molecules. In other embodiments, the maximum value of peptide or protein expressed by a given number of RNA molecules does not need to be higher than the maximum value of peptide or protein expressed by the same number of RNA molecules, however, the average amount of peptide or protein expressed by the given number of RNA molecules within a given period of time may be higher than the average amount of peptide or protein expressed by the same number of RNA molecules. The latter cases are referred to herein as "higher level of expression" or "increased level of expression" and relate to higher maximum values of expression and/or higher average values of expression. Alternatively or additionally, terms such as "enhancement of expression", "enhanced expression" or "increased expression" mean in the context of the present teaching also that the time in which peptide or protein is expressed by RNA molecules may be longer than the time in which the peptide or protein is expressed by the same RNA molecules. Thus, in one embodiment, terms such as "enhancement of expression", "enhanced expression" or "increased expression" mean in the context of the present teaching also that the amount of peptide or protein expressed by a given number of RNA molecules is higher than the amount of peptide or protein expressed by the same number of RNA molecules since the period of time in which the RNA is stably present and expressed is longer than the period of time in which the same number of RNA molecules is stably present and expressed. These cases are referred to herein also as "increased duration of expression". Preferably, such longer time periods refer to expression for at least 48 h, preferably for at least 72 h, more preferably for at least 96 h, in particular for at least 120 h or even longer following introduction of RNA or following the first introduction (e.g. in case of repeated transfections) of RNA into a cell.

The level of expression and/or duration of expression of RNA may be determined by measuring the amount, such as the total amount expressed and/or the amount expressed in a given time period, and/or the time of expression of the peptide or protein encoded by the RNA, for example, by using an ELISA procedure, an immunohistochemistry procedure, a quantitative image analysis procedure, a Western Blot, mass spectrometry, a quantitative immunohistochemistry procedure, or an enzymatic assay.

According to the teaching, the term "RNA encoding" with respect to a particular peptide or protein means that the RNA, if present in the appropriate environment, preferably within a cell, can be expressed to produce said peptide or protein.

Preferably, RNA according to the teaching is able to interact with the cellular translation machinery to provide the peptide or protein it encodes.

In particular embodiments, the RNA according to the teaching comprises a single type of RNA molecule, a population of different RNA molecules, e.g. a mixture of different RNA molecules optionally encoding different peptides and/or proteins, whole-cell RNA, a RNA library, or a portion of thereof, e.g. a library of RNA molecules expressed in a particular cell type, such as undifferentiated cells, in particular stem cells such as embryonic stem cells, or a fraction of the library of RNA molecules such as RNA with enriched expression in undifferentiated cells, in particular stem cells such as embryonic stem cells relative to differentiated cells. Thus, according to the teaching, the term "RNA" may include a mixture of RNA molecules, whole-cell RNA or a fraction thereof, which may be obtained by a process comprising the isolation of RNA from cells and/or by recombinant means, in particular by *in vitro* transcription.

The RNA used according to the present teaching may have a known composition (in this embodiment it is preferably known which peptides or proteins are being expressed by the RNA) or the composition of the RNA may be partially or entirely unknown. Alternatively, the RNA used according to the present teaching may have a known function or the function of the RNA may be partially or entirely unknown.

Preferably, according to the teaching, the RNA to be expressed in a cell is introduced into said cell, either *in vitro* or *in vivo,* preferably *in vitro.* RNA may be introduced into a cell either prior to, after and/or simultaneously with providing a virus-derived factor comprising *Toscana virus* NSs protein or a functional variant of *Toscana virus* NSs protein to the cell. Preferably, a virus-derived factor comprising *Toscana virus* NSs protein or a functional variant of *Toscana virus* NSs protein is provided as long as expression of the RNA in the cell is desired. In one embodiment of the methods according to the teaching, the RNA that is to be introduced into a cell is obtained by *in vitro* transcription of an appropriate DNA template.

According to the teaching, RNA may be introduced into cells either *in vitro* or *in vivo,* preferably *in vitro.* The cells into which the RNA is introduced *in vitro* may, preferably following expression of the RNA *in vitro* by the methods of the teaching, be administered to a patient.

The term "transfection" relates to the introduction of nucleic acids, in particular RNA, into a cell. For purposes of the present teaching, the term "transfection" also includes the introduction of a nucleic acid into a cell or the uptake of a nucleic acid by such cell, wherein the cell may be present in a subject, e.g., a patient. Thus, according to the present teaching, a cell for transfection of a nucleic acid described herein can be present *in vitro* or *in vivo,* e.g. the cell can form part of an organ, a tissue and/or an organism of a patient. According to the teaching, transfection can be transient or stable. For some applications of transfection, it is sufficient if the transfected genetic material is only transiently expressed. Since the nucleic acid introduced in the transfection process is usually not integrated into the nuclear genome, the foreign nucleic acid will be diluted through mitosis or degraded. Cells allowing episomal amplification of nucleic acids greatly reduce the rate of dilution. If it is desired that the transfected nucleic acid actually remains in the genome of the cell and its daughter cells, a stable transfection must occur. RNA can be transfected into cells to transiently express its coded protein.

According to the present teaching, any technique useful for introducing, i.e. transferring or transfecting, nucleic acids into cells may be used. Preferably, RNA is transfected into cells by standard techniques. Such techniques include electroporation, lipofection and microinjection. In one particularly preferred embodiment of the present teaching, RNA is introduced into cells by electroporation. Electroporation or electropermeabilization relates to a significant increase in the electrical conductivity and permeability of the cell plasma membrane caused by an externally applied electrical field. It is usually used in molecular biology as a way of introducing some substance into a cell. According to the teaching it is preferred that introduction of nucleic acid encoding a protein or peptide into cells results in expression of said protein or peptide.

A cell may express the encoded peptide or protein intracellularly (e.g. in the cytoplasm and/or in the nucleus), may secrete the encoded peptide or protein, or may express it on the surface.

According to the present teaching, a cell can be an isolated cell or it can form part of an organ, a tissue and/or an organism. According to the present teaching, the administration of a nucleic acid is either achieved as naked nucleic acid or in combination with an administration reagent. Preferably, administration of nucleic acids is in the form of naked nucleic acids. Preferably, the RNA is administered in combination with stabilizing substances such as RNase inhibitors. The present teaching also envisions the repeated introduction of nucleic acids into cells to allow sustained expression for extended time periods.

According to the teaching, nucleic acids may be directed to particular cells. In such embodiments, a carrier used for administering a nucleic acid to a cell (e.g. a retrovirus or a liposome) may have a bound targeting molecule. For example, a molecule such as an antibody specific to a surface membrane protein on the target cell, or a ligand for a receptor on the target cell may be incorporated into or bound to the nucleic acid carrier. If administration of a nucleic acid by liposomes is desired, proteins binding to a surface membrane protein associated with endocytosis may be incorporated into the liposome formulation in order to enable targeting and/or absorption. Such proteins include capsid proteins or fragments thereof which are specific to a particular cell type, antibodies to proteins that are internalized, proteins targeting an intracellular site, and the like.

According to the teaching, the term "introducing RNA into cells" includes and preferably relates to embodiments wherein the RNA is only introduced into a portion of the cells and a portion of the cells remains un-transfected. If more than one RNA molecule is to be introduced into cells, e.g., more than one RNA molecule expressing a functional set of reprogramming factors or RNA encoding a protein of interest and RNA encoding a virus-derived factor comprising *Toscana virus* NSs protein or a functional variant of *Toscana virus* NSs protein, the term includes and preferably relates to embodiments wherein all RNA molecules are only introduced into a portion of the cells and a portion of the cells remains un-transfected or remains transfected by not all of said RNA molecules. In any case, if according to the teaching more than one RNA molecule is to be introduced into cells, e.g., more than one RNA molecule expressing a functional set of reprogramming factors, the aim is to introduce all RNA molecules into a cell, e.g., to express a functional set of reprogramming factors within a single cell.

Terms such as "enhancement of cell viability", "enhanced cell viability" or "increased cell viability" mean in the context of the present teaching that the amount of viable or living cells under certain conditions is higher than the amount of viable or living cells under other conditions, wherein cultivation is performed under the same conditions except the condition which results in the enhanced or increased cell viability, such as providing a virus-derived factor comprising *Toscana virus* NSs protein or a functional variant of *Toscana virus* NSs protein to a cell versus not providing a virus-derived factor comprising *Toscana virus* NSs protein or a functional variant of *Toscana virus* NSs protein to a cell. In this context, "same conditions" refer to a situation wherein the same cells are used, the cells are cultured under the same conditions (except the condition which results in the enhanced or increased cell viability) and the cell viability is measured by the same means. "Same conditions" also encompasses the introduction or repetitive introduction of RNA into cells.

The term "cell" or "host cell" preferably relates to an intact cell, i.e. a cell with an intact membrane that has not released its normal intracellular components such as enzymes, organelles, or genetic material. An intact cell preferably is a viable cell, i.e. a living cell capable of carrying out its normal metabolic functions. Preferably said term relates according to the teaching to any cell which can be transformed or transfected with an exogenous nucleic acid. The term "cell" includes according to the teaching prokaryotic cells (e.g., E. coli) or eukaryotic cells. Mammalian cells are particularly preferred, such as cells from humans, mice, hamsters, pigs, goats, and primates. The cells may be derived from a multiplicity of tissue types and comprise primary cells and cell lines. Specific examples include keratinocytes, peripheral blood leukocytes, bone marrow stem cells and embryonic stem cells. In other embodiments, the host cell is an antigen-presenting cell, in particular a dendritic cell, a monocyte or a macrophage. In one embodiment, the cell is a somatic cell as described herein. In one embodiment, the cell is a cell having a barrier function. Preferably, the cell is a fibroblast such as a fibroblast described herein, a keratinocyte, an epithelial cell, or an endothelial cell such as an endothelial cell of the heart, an endothelial cell of the lung, or an umbilical vein endothelial cell. Preferably, the cell is a human cell.

A fibroblast is a type of cell that synthesizes the extracellular matrix and collagen and plays a critical role in wound healing. The main function of fibroblasts is to maintain the structural integrity of connective tissues by continuously secreting precursors of the extracellular matrix. Fibroblasts are the most common cells of connective tissue in animals. Fibroblasts are morphologically heterogeneous with diverse appearances depending on their location and activity.

Keratinocytes are the predominant cell type in the epidermis, the outermost layer of the human skin. The primary function of keratinocytes is the formation of the keratin layer that protects the skin and the underlying tissue from environmental damage such as heat, UV radiation and water loss.

Epithelium is a tissue composed of cells that line the cavities and surfaces of structures throughout the body. Many glands are also formed from epithelial tissue. It lies on top of connective tissue, and the two layers are separated by a basement membrane. In humans, epithelium is classified as a primary body tissue, the other ones being connective tissue, muscle tissue and nervous tissue. Functions of epithelial cells include secretion, selective absorption, protection, transcellular transport and detection of sensation.

The endothelium is the thin layer of cells that lines the interior surface of blood vessels, forming an interface between circulating blood in the lumen and the rest of the vessel wall. These cells are called endothelial cells. Endothelial cells line the entire circulatory system, from the heart to the smallest capillary. Endothelial tissue is a specialized type of epithelium tissue.

The term "recombinant" in the context of the present teaching means "made through genetic engineering". Preferably, a "recombinant object" such as a recombinant cell in the context of the present teaching is not occurring naturally.

The term "naturally occurring" as used herein refers to the fact that an object can be found in nature. For example, a peptide or nucleic acid that is present in an organism (including viruses) and can be isolated from a source in nature and which has not been intentionally modified by man in the laboratory is naturally occurring.

The RNA to be expressed in a cell encodes one or more peptides or proteins of interest. Accordingly, RNA may contain one or more open reading frames (ORFs) encoding one or more peptides or proteins of interest. The RNA to be expressed in a cell may encode a variety of peptides or proteins of interest, in particular peptides or proteins that are of interest when expressed in a particular cell type since they can perform a particular function when expressed in said cell type.

According to the present teaching, the term "peptide" comprises oligo- and polypeptides and refers to substances which comprise two or more, preferably 3 or more, preferably 4 or more, preferably 6 or more, preferably 8 or more, preferably 10 or more, preferably 13 or more, preferably 16 or more, preferably 20 or more, and up to preferably 50, preferably 100 or preferably 150, consecutive amino acids linked to one another via peptide bonds. The term "protein" refers to large peptides, preferably peptides having at least 151 amino acids, but the terms "peptide" and "protein" are used herein usually as synonyms.

The terms "peptide" and "protein" comprise according to the teaching substances which contain not only amino acid components but also non-amino acid components such as sugars and phosphate structures, and also comprise substances containing bonds such as ester, thioether or disulfide bonds.

The RNA according to the present teaching may encode a single peptide or protein, or multiple peptides or proteins. Multiple peptides or proteins can be encoded as a single polypeptide (fusion polypeptide) or as separate peptides or proteins. A polyprotein or fusion polypeptide may comprise individual polypeptides separated by an optionally autocatalytic protease cleavage site (e.g. foot-and-mouth disease virus 2A protein), or an intein.

According to the teaching, in one embodiment, RNA comprises or consists of pharmaceutically active RNA. A "pharmaceutically active RNA" may be RNA that encodes a pharmaceutically active peptide or protein.

A "pharmaceutically active peptide or protein" has a positive or advantageous effect on the condition or disease state of a subject when administered to the subject in a therapeutically effective amount. Preferably, a pharmaceutically active peptide or protein has curative or palliative properties and may be administered to ameliorate, relieve, alleviate, reverse, delay onset of or lessen the severity of one or more symptoms of a disease or disorder. A pharmaceutically active peptide or protein may have prophylactic properties and may be used to delay the onset of a disease or to lessen the severity of such disease or pathological condition. The term "pharmaceutically active peptide or protein" includes entire proteins or polypeptides, and can also refer to pharmaceutically active fragments thereof. It can also include pharmaceutically active analogs of a peptide or protein. The term "pharmaceutically active peptide or protein" includes peptides and proteins that are antigens, i.e., the peptide or protein elicits an immune response in a subject which may be therapeutic or partially or fully protective.

In one embodiment, the pharmaceutically active peptide or protein is or comprises an immunologically active compound or an antigen or an epitope.

According to the teaching, the term "immunologically active compound" relates to any compound altering an immune response, preferably by inducing and/or suppressing maturation of immune cells, inducing and/or suppressing cytokine biosynthesis, and/or altering humoral immunity by stimulating antibody production by B cells. In one embodiment, the immune response involves stimulation of an antibody response (usually including immunoglobulin G (IgG)). Immunologically active compounds possess potent immunostimulating activity including, but not limited to, antiviral and antitumor activity, and can also down-regulate other aspects of the immune response, for example shifting the immune response away from a TH2 immune response, which is useful for treating a wide range of TH2 mediated diseases.

According to the teaching, the term "antigen" or "immunogen" covers any substance that will elicit an immune response. In particular, an "antigen" relates to any substance that reacts specifically with antibodies or T-lymphocytes (T-cells). According to the present teaching, the term "antigen" comprises any molecule which comprises at least one epitope. Preferably, an antigen in the context of the present teaching is a molecule which, optionally after processing, induces an immune reaction, which is preferably specific for the antigen. According to the present teaching, any suitable antigen may be used, which is a candidate for an immune reaction, wherein the immune reaction may be both a humoral as well as a cellular immune reaction. In the context of the embodiments of the present teaching, the antigen is preferably presented by a cell, preferably by an antigen presenting cell, in the context of MHC molecules, which results in an immune reaction against the antigen. An antigen is preferably a product which corresponds to or is derived from a naturally occurring antigen. Such naturally occurring antigens may include or may be derived from allergens, viruses, bacteria, fungi, parasites and other infectious agents and pathogens or an antigen may also be a tumor antigen. According to the present teaching, an antigen may correspond to a naturally occurring product, for example, a viral protein, or a part thereof. In preferred embodiments, the antigen is a surface polypeptide, i.e. a polypeptide naturally displayed on the surface of a cell, a pathogen, a bacterium, a virus, a fungus, a parasite, an allergen, or a tumor. The antigen may elicit an immune response against a cell, a pathogen, a bacterium, a virus, a fungus, a parasite, an allergen, or a tumor.

The term "pathogen" refers to pathogenic biological material capable of causing disease in an organism, preferably a vertebrate organism. Pathogens include microorganisms such as bacteria, unicellular eukaryotic organisms (protozoa), fungi, as well as viruses.

The terms "epitope", "antigen peptide", "antigen epitope", "immunogenic peptide" and "MHC binding peptide" are used interchangeably herein and refer to an antigenic determinant in a molecule such as an antigen, i.e., to a part in or fragment of an immunologically active compound that is recognized by the immune system, for example, that is recognized by a T cell, in particular when presented in the context of MHC molecules. An epitope of a protein preferably comprises a continuous or discontinuous portion of said protein and is preferably between 5 and 100, preferably between 5 and 50, more preferably between 8 and 30, most preferably between 10 and 25 amino acids in length, for example, the epitope may be preferably 9, 10, 11, 12, 13, 14, 15, 16, 17, 18, 19, 20, 21, 22, 23, 24, or 25 amino acids in length. According to the teaching an epitope may bind to MHC molecules such as MHC molecules on the surface of a cell and thus, may be a "MHC binding peptide" or "antigen peptide". The term "major histocompatibility complex" and the abbreviation "MHC" include MHC class I and MHC class II molecules and relate to a complex of genes which is present in all vertebrates. MHC proteins or molecules are important for signaling between lymphocytes and antigen presenting cells or diseased cells in immune reactions, wherein the MHC proteins or molecules bind peptides and present them for recognition by T cell receptors. The proteins encoded by the MHC are expressed on the surface of cells, and display both self-antigens (peptide fragments from the cell itself) and non-self-antigens (e.g., fragments of invading microorganisms) to a T cell. Preferred such immunogenic portions bind to an MHC class I or class II molecule. As used herein, an immunogenic portion is said to "bind to" an MHC class I or class II molecule if such binding is detectable using any assay known in the art. The term "MHC binding peptide" relates to a peptide which binds to an MHC class I and/or an MHC class II molecule. In the case of class I MHC/peptide complexes, the binding peptides are typically 8-10 amino acids long although longer or shorter peptides may be effective. In the case of class II MHC/peptide complexes, the binding peptides are typically 10-25 amino acids long and are in particular 13-18 amino acids long, whereas longer and shorter peptides may be effective.

In one embodiment, the protein of interest according to the present teaching comprises an epitope suitable for vaccination of a target organism. A person skilled in the art will know that one of the principles of immunobiology and vaccination is based on the fact that an immunoprotective reaction to a disease is produced by immunizing an organism with an antigen, which is immunologically relevant with respect to the disease to be treated. According to the present teaching, an antigen is selected from the group comprising a self-antigen and non-self-antigen. A non-self-antigen is preferably a bacterial antigen, a virus antigen, a fungus antigen, an allergen or a parasite antigen. It is preferred that the antigen comprises an epitope that is capable of eliciting an immune response in a target organism. For example, the epitope may elicit an immune response against a bacterium, a virus, a fungus, a parasite, an allergen, or a tumor.

In some embodiments the non-self-antigen is a bacterial antigen. In some embodiments, the antigen elicits an immune response against a bacterium which infects animals, including birds, fish and mammals, including domesticated animals. Preferably, the bacterium against which the immune response is elicited is a pathogenic bacterium.

In some embodiments the non-self-antigen is a virus antigen. A virus antigen may for example be a peptide from a virus surface protein, e.g. a capsid polypeptide or a spike polypeptide. In some embodiments, the antigen elicits an immune response against a virus which infects animals, including birds, fish and mammals, including domesticated animals. Preferably, the virus against which the immune response is elicited is a pathogenic virus.

In some embodiments the non-self-antigen is a polypeptide or a protein from a fungus. In some embodiments, the antigen elicits an immune response against a fungus which infects animals, including birds, fish and mammals, including domesticated animals. Preferably, the fungus against which the immune response is elicited is a pathogenic fungus.

In some embodiments the non-self-antigen is a polypeptide or protein from a unicellular eukaryotic parasite. In some embodiments, the antigen elicits an immune response against a unicellular eukaryotic parasite, preferably a pathogenic unicellular eukaryotic parasite. Pathogenic unicellular eukaryotic parasites may be e.g. from the genus Plasmodium, e.g. P. falciparum, P. vivax, P. malariae or P. ovale, from the genus Leishmania, or from the genus Trypanosoma, e.g. T. cruzi or T. brucei.

In some embodiments the non-self-antigen is an allergenic polypeptide or an allergenic protein. An allergenic protein or allergenic polypeptide is suitable for allergen immunotherapy, also known as hypo-sensitization.

In some embodiments the antigen is a self-antigen, particularly a tumor antigen. Tumor antigens and their determination are known to the skilled person.

In the context of the present teaching, the term "tumor antigen" or "tumor-associated antigen" relates to proteins that are under normal conditions specifically expressed in a limited number of tissues and/or organs or in specific developmental stages, for example, the tumor antigen may be under normal conditions specifically expressed in stomach tissue, preferably in the gastric mucosa, in reproductive organs, e.g., in testis, in trophoblastic tissue, e.g., in placenta, or in germ line cells, and are expressed or aberrantly expressed in one or more tumor or cancer tissues. In this context, "a limited number" preferably means not more than 3, more preferably not more than 2. The tumor antigens in the context of the present teaching include, for example, differentiation antigens, preferably cell type specific differentiation antigens, i.e., proteins that are under normal conditions specifically expressed in a certain cell type at a certain differentiation stage, cancer/testis antigens, i.e., proteins that are under normal conditions specifically expressed in testis and sometimes in placenta, and germ line specific antigens. In the context of the present teaching, the tumor antigen is preferably associated with the cell surface of a cancer cell and is preferably not or only rarely expressed in normal tissues. Preferably, the tumor antigen or the aberrant expression of the tumor antigen identifies cancer cells. In the context of the present teaching, the tumor antigen that is expressed by a cancer cell in a subject, e.g., a patient suffering from a cancer disease, is preferably a self-protein in said subject. In preferred embodiments, the tumor antigen in the context of the present teaching is expressed under normal conditions specifically in a tissue or organ that is non-essential, i.e., tissues or organs which when damaged by the immune system do not lead to death of the subject, or in organs or structures of the body which are not or only hardly accessible by the immune system. Preferably, the amino acid sequence of the tumor antigen is identical between the tumor antigen which is expressed in normal tissues and the tumor antigen which is expressed in cancer tissues.

Examples for tumor antigens that may be useful in the present teaching are p53, ART-4, BAGE, beta-catenin/m, Bcr-abL CAMEL, CAP-1, CASP-8, CDC27/m, CDK4/m, CEA, the cell surface proteins of the claudin family, such as CLAUDIN-6, CLAUDIN-18.2 and CLAUDIN-12, c-MYC, CT, Cyp-B, DAM, ELF2M, ETV6-AML1, G250, GAGE, GnT-V, Gap100, HAGE, HER-2/neu, HPV-E7, HPV-E6, HAST-2, hTERT (or hTRT), LAGE, LDLR/FUT, MAGE-A, preferably MAGE-A1, MAGE-A2, MAGE-A3, MAGE-A4, MAGE-A5, MAGE-A6, MAGE-A7, MAGE-A8, MAGE-A9, MAGE-A10, MAGE-A11, or MAGE-A12, MAGE-B, MAGE-C, MART-1/Melan-A, MC1R, Myosin/m, MUC1, MUM-1, -2, -3, NA88-A, NF1, NY-ESO-1, NY-BR-1, p190 minor BCR-abL, Pm1/RARa, PRAME, proteinase 3, PSA, PSM, RAGE, RU1 or RU2, SAGE, SART-1 or SART-3, SCGB3A2, SCP1, SCP2, SCP3, SSX, SURVIVIN, TEL/AML1, TPI/m, TRP-1, TRP-2, TRP-2/INT2, TPTE and WT. Particularly preferred tumor antigens include CLAUDIN-18.2 (CLDN18.2) and CLAUDIN-6 (CLDN6).

If, according to the present teaching, it is desired to induce or enhance an immune response by using RNA as described herein, antigens or epitopes encoded by the RNA or procession products thereof may be presented by major histocompatibility complex (MHC) proteins expressed on antigen presenting cells such as dendritic cell. The MHC peptide complex can then be recognized by immune cells such as T cells leading to their activation. Thus, preferred cells used in connection with RNA encoding antigenic proteins or peptides are antigen presenting cells such as dendritic cell.

Thus, the present teaching envisions embodiments wherein RNA encoding an antigen is introduced into antigen-presenting cells *ex vivo,* e.g., antigen-presenting cells taken from a patient, to express the antigen, and the antigen-presenting cells, optionally clonally propagated *ex vivo,* are transplanted into a patient, e.g., into the same patient. Transfected cells may be introduced into a patient using any means known in the art, preferably in sterile form by intravenous, intracavitary, intraperitoneal or intratumor administration.

In one embodiment, RNA that codes for an antigen may be administered to a mammal, in particular if treating a mammal having a disease involving the antigen is desired. The same RNA may contain an open reading frame encoding a virus-derived factor comprising *Toscana virus* NSs protein or a functional variant of *Toscana virus* NSs protein or RNA containing an open reading frame encoding a virus-derived factor comprising *Toscana virus* NSs protein or a functional variant of *Toscana virus* NSs protein may be additionally administered. The RNA is taken up into the mammal's antigen-presenting cells (monocytes, macrophages, dendritic cells or other cells). An antigenic translation product of the RNA is formed and the product is displayed on the surface of the cells for recognition by T cells.

The methods of the teaching may involve an antigen presenting cell for expressing the RNA encoding the antigen. To this end, the methods of the teaching may involve introduction of RNA encoding antigens into antigen presenting cells such as dendritic cells. For transfection of antigen presenting cells such as dendritic cells a pharmaceutical composition comprising RNA encoding the antigen may be used. A delivery vehicle that targets the RNA to a dendritic or other antigen presenting cell may be administered to a patient, resulting in transfection that occurs *in vivo.*

In some embodiments, it is not required that the pharmaceutically active peptide or protein is an antigen that elicits an immune response. Suitable pharmaceutically active proteins or peptides may be selected from the group consisting of cytokines and immune system proteins such as immunologically active compounds (e.g., interleukins, colony stimulating factor (CSF), granulocyte colony stimulating factor (G-CSF), granulocyte-macrophage colony stimulating factor (GM-CSF), erythropoietin, tumor necrosis factor (TNF), interferons, integrins, addressins, seletins, homing receptors, T cell receptors, immunoglobulins), hormones (insulin, thyroid hormone, catecholamines, gonadotrophines, trophic hormones, prolactin, oxytocin, dopamine, bovine somatotropin, leptins and the like), growth hormones (e.g., human grown hormone), growth factors (e.g., epidermal growth factor, nerve growth factor, insulin-like growth factor and the like), growth factor receptors, enzymes (tissue plasminogen activator, streptokinase, cholesterol biosynthetic or degradative, steriodogenic enzymes, kinases, phosphodiesterases, methylases, de-methylases, dehydrogenases, cellulases, proteases, lipases, phospholipases, aromatases, cytochromes, adenylate or guanylaste cyclases, neuramidases and the like), receptors (steroid hormone receptors, peptide receptors), binding proteins (growth hormone or growth factor binding proteins and the like), transcription and translation factors, tumor growth suppressing proteins (e.g., proteins which inhibit angiogenesis), structural proteins (such as collagen, fibroin, fibrinogen, elastin, tubulin, actin, and myosin), blood proteins (thrombin, serum albumin, Factor VII, Factor VIII, insulin, Factor IX, Factor X, tissue plasminogen activator, protein C, von Willebrand factor, antithrombin III, glucocerebrosidase, erythropoietin granulocyte colony stimulating factor (GCSF) or modified Factor VIII, anticoagulants and the like. In one embodiment, the pharmaceutically active protein according to the teaching is a cytokine which is involved in regulating lymphoid homeostasis, preferably a cytokine which is involved in and preferably induces or enhances development, priming, expansion, differentiation and/or survival of T cells. In one embodiment, the cytokine is an interleukin, e.g. IL-2, IL-7, IL-12, IL-15, or IL-21.

Cells expressing a peptide or protein as described herein may be used in the therapeutic or prophylactic treatment of various diseases, in particular diseases in which provision of said peptide or protein results in a therapeutic or prophylactic effect. For example, expression of an antigen which is derived from a virus may be useful in the treatment of a viral disease caused by said virus. Expression of a tumor antigen may be useful in the treatment of a cancer disease wherein cancer cells express said tumor antigen.

The term "disease" refers to an abnormal condition that affects the body of an individual. A disease is often construed as a medical condition associated with specific symptoms and signs. A disease may be caused by factors originally from an external source, such as infectious disease, or it may be caused by internal dysfunctions, such as autoimmune diseases.

According to the teaching, the term "disease" also refers to cancer diseases. The terms "cancer disease" or "cancer" (medical term: malignant neoplasm) refer to a class of diseases in which a group of cells display uncontrolled growth (division beyond the normal limits), invasion (intrusion on and destruction of adjacent tissues), and sometimes metastasis (spread to other locations in the body via lymph or blood). These three malignant properties of cancers differentiate them from benign tumors, which are self-limited, and do not invade or metastasize. Most cancers form a tumor, i.e. a swelling or lesion formed by an abnormal growth of cells (called neoplastic cells or tumor cells), but some, like leukemia, do not. Examples of cancers include, but are not limited to, carcinoma, lymphoma, blastoma, sarcoma, glioma and leukemia. More particularly, examples of such cancers include bone cancer, blood cancer, lung cancer, liver cancer, pancreatic cancer, skin cancer, cancer of the head or neck, cutaneous or intraocular malignant melanoma, uterine cancer, ovarian cancer, rectal cancer, cancer of the anal region, stomach cancer, colon cancer, breast cancer, prostate cancer, uterine cancer, carcinoma of the sexual and reproductive organs, Hodgkin's disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the renal pelvis, neoplasms of the central nervous system (CNS), neuroectodermal cancer, spinal axis tumors, glioma, meningioma, and pituitary adenoma. The term "cancer" according to the teaching also comprises cancer metastases.

The term "infectious disease" refers to any disease which can be transmitted from individual to individual or from organism to organism, and is caused by a microbial agent (e.g. common cold). Examples of infectious diseases include viral infectious diseases, such as AIDS (HIV), hepatitis A, B or C, herpes, herpes zoster (chicken-pox), German measles (rubella virus), yellow fever, dengue etc. flaviviruses, influenza viruses, hemorrhagic infectious diseases (Marburg or Ebola viruses), and severe acute respiratory syndrome (SARS), bacterial infectious diseases, such as Legionnaire's disease (*Legionella*), sexually transmitted diseases (e.g. chlamydia or gonorrhea), gastric ulcer (*Helicobacter*), cholera (*Vibrio*), tuberculosis, diphtheria, infections by *E.coli, Staphylococci, Salmonella* or *Streptococci* (tetanus); infections by protozoan pathogens such as malaria, sleeping sickness, leishmaniasis; toxoplasmosis, i.e. infections by Plasmodium, Trypanosoma, Leishmania and Toxoplasma; or fungal infections, which are caused e.g. by *Cryptococcus neoformans, Histoplasma capsulatum, Coccidioides immitis, Blastomyces dermatitidis* or *Candida albicans.*

The term "autoimmune disease" refers to any disease in which the body produces an immunogenic (i.e. immune system) response to some constituent of its own tissue. In other words, the immune system loses its ability to recognize some tissue or system within the body as self and targets and attacks it as if it were foreign. Autoimmune diseases can be classified into those in which predominantly one organ is affected (e.g. hemolytic anemia and anti-immune thyroiditis), and those in which the autoimmune disease process is diffused through many tissues (e.g. systemic lupus erythematosus). For example, multiple sclerosis is thought to be caused by T cells attacking the sheaths that surround the nerve fibers of the brain and spinal cord. This results in loss of coordination, weakness, and blurred vision. Autoimmune diseases are known in the art and include, for instance, Hashimoto's thyroiditis, Grave's disease, lupus, multiple sclerosis, rheumatic arthritis, hemolytic anemia, anti-immune thyroiditis, systemic lupus erythematosus, celiac disease, Crohn's disease, colitis, diabetes, scleroderma, psoriasis, and the like.

The RNA described herein is also useful in expressing a T cell receptor or an artificial T cell receptor in a cell, in particular an immune effector cell such as a T cell, *in vitro* or *in vivo.* Thus, in one embodiment, the peptide or protein encoded by RNA described herein is one or more chains of a T cell receptor or of an artificial T cell receptor.

Cells engineered to express such T cell receptor or artificial T cell receptor are useful in the treatment of diseases characterized by expression of antigens such as antigens described herein bound by the T cell receptor or artificial T cell receptor, in particular diseases as described herein. Adoptive cell transfer therapy with engineered T cells expressing T cell receptors or artificial T cell receptors is a promising therapeutic. For example, patient's T cells may be genetically engineered (genetically modified) to express T cell receptors or artificial T cell receptors specifically directed towards antigens on the patient's diseased cells, and then infused back into the patient.

Thus, in a further aspect, the present teaching provides a method of producing an immunoreactive cell comprising the step of introducing into a T cell or a progenitor thereof one or more RNA molecules encoding the chains of a T cell receptor or the chain(s) of an artificial T cell receptor, and providing a virus-derived factor comprising *Toscana virus* NSs protein or a functional variant of *Toscana virus* NSs protein to the cell.

The term "immunoreactive cell" or "immune effector cell" in the context of the present teaching relates to a cell which exerts effector functions during an immune reaction. An "immunoreactive cell" preferably is capable of binding an antigen such as an antigen expressed on the surface of a cell or presented on the surface of a cell in the context of MHC molecules and mediating an immune response. For example, such cells secrete cytokines and/or chemokines, kill microbes, secrete antibodies, recognize infected or cancerous cells, and optionally eliminate such cells. For example, immunoreactive cells comprise T cells (cytotoxic T cells, helper T cells, tumor infiltrating T cells), B cells, natural killer cells, neutrophils, macrophages, and dendritic cells. Preferably, in the context of the present teaching, "immunoreactive cells" are T cells, preferably CD4+ and/or CD8+ T cells. According to the teaching, the term "immunoreactive cell" also includes a cell which can mature into an immune cell (such as T cell, in particular T helper cell, or cytolytic T cell) with suitable stimulation. Immunoreactive cells comprise CD34+ hematopoietic stem cells, immature and mature T cells and immature and mature B cells. The differentiation of T cell precursors into a cytolytic T cell, when exposed to an antigen, is similar to clonal selection of the immune system.

A majority of T cells have a T cell receptor (TCR) existing as a complex of several proteins. The actual T cell receptor is composed of two separate peptide chains, which are produced from the independent T cell receptor alpha and beta (TCRα and TCRβ) genes and are called α- and β-TCR chains. γδ T cells (gamma delta T cells) represent a small subset of T cells that possess a distinct T cell receptor (TCR) on their surface. However, in γδ T cells, the TCR is made up of one γ-chain and one δ-chain. This group of T cells is much less common (2% of total T cells) than the αβ T cells. In a preferred embodiment of the teaching, a T cell receptor comprises α- and β-TCR chains. The RNA encoding α- and β-chains of a T cell receptor may be contained on separate nucleic acid molecules, i.e., RNA molecules, or alternatively, on a single RNA molecule. Accordingly, expression of a T cell receptor in a cell requires co-transfection of separate RNA molecules encoding the different T cell receptor chains or transfection of only one type of RNA molecule encoding the different T cell receptor chains.

Engineered receptors, termed "artificial T cell receptor", "chimeric antigen receptor (CAR)" or "chimeric T cell receptor" herein, have been produced, which confer an arbitrary specificity such as the specificity of a monoclonal antibody onto an immune effector cell such as a T cell. Such T cells do not necessarily require processing and presentation of an antigen for recognition of the target cell but rather may recognize any antigen present on a target cell. According to the teaching, an artificial T cell receptor may comprise an antigen binding domain, e.g., antigen-binding portions of antibodies and T cell receptors such as single-chain variable fragments (scFv), a T cell signaling domain, e.g., the endodomain of CD3-zeta, and optionally one or more co-stimulation domains, e.g., CD28, CD137 (4-1 BB), CD134 (OX40), and CD278 (ICOS).

The RNA described herein is also useful in reprogramming or de-differentiating somatic cells into stem-like cells, i.e. cells having stem cell characteristics, *in vitro* or *in vivo.* This may involve the transient expression of reprogramming factors *in vitro* or *in vivo* in order to initiate reprogramming or de-differentiation processes in cells. Thus, in one embodiment, the peptide or protein encoded by RNA described herein is a factor allowing the reprogramming of somatic cells to cells having stem cell characteristics. Stem-like cells can be provided according to the teaching without generating embryos or fetuses. De-differentiation of somatic cells to cells having stem cell characteristics, in particular pluripotency, can be effected by introducing RNA encoding factors inducing the de-differentiation of somatic cells into the somatic cells (also termed reprogramming transcription factors (rTF) or reprogramming factors) and culturing the somatic cells allowing the cells to de-differentiate. After being de-differentiated, the cells could be induced to re-differentiate into the same or a different somatic cell type such as neuronal, hematopoietic, muscle, epithelial, and other cell types. Thus, such stem-like cells have medical applications for treatment of degenerative diseases by "cell therapy" and may be utilized in novel therapeutic strategies in the treatment of cardiac, neurological, endocrinological, vascular, retinal, dermatological, muscular-skeletal disorders, and other diseases.

Accordingly, the teaching also relates to a method for providing cells having stem cell characteristics comprising the steps of (i) providing a cell population comprising somatic cells, (ii) providing a virus-derived factor comprising *Toscana virus* NSs protein or a functional variant of *Toscana virus* NSs protein to the somatic cells, (iii) introducing RNA encoding one or more reprogramming factors into the somatic cells, and (iv) allowing the development of cells having stem cell characteristics.

In one embodiment, the one or more reprogramming factors comprise OCT4 and SOX2. The one or more reprogramming factors may further comprise KLF4 and/or c-MYC and/or NANOG and/or LIN28. In one embodiment, the one or more reprogramming factors comprise OCT4, SOX2, KLF4 and c-MYC and may further comprise LIN28 and optionally NANOG. In one embodiment, the one or more reprogramming factors comprise OCT4, SOX2, NANOG and LIN28.

In one embodiment, the method further comprises the step of culturing the somatic cells in the presence of at least one histone deacetylase inhibitor, wherein the at least one histone deacetylase inhibitor preferably comprises valproic acid, sodium butyrate, trichostatin A and/or scriptaid.

In one embodiment, the step of allowing the development of cells having stem cell characteristics comprises culturing the somatic cells under embryonic stem cell culture conditions.

In one embodiment, the stem cell characteristics comprise an embryonic stem cell morphology.

In one embodiment, the cells having stem cell characteristics have normal karyotypes, express telomerase activity, express cell surface markers that are characteristic for embryonic stem cells and/or express genes that are characteristic for embryonic stem cells.

In one embodiment, the cells having stem cell characteristics exhibit a pluripotent state.

In one embodiment, the cells having stem cell characteristics have the developmental potential to differentiate into advanced derivatives of all three primary germ layers.

In one embodiment, the somatic cells are fibroblasts such as lung fibroblasts, foreskin fibroblasts or dermal fibroblasts. Preferably, the somatic cells are human cells.

In one embodiment, the RNA is introduced into the somatic cells by electroporation or lipofection. In one embodiment, the RNA is introduced into the somatic cells repetitively.

In one embodiment, introduction of RNA encoding reprogramming factors as disclosed herein into somatic cells results in expression of said factors for an extended period of time, preferably for at least 10 days, preferably for at least 11 days and more preferably for at least 12 days. To achieve such long term expression, RNA is preferably periodically (i.e. repetitively) introduced into the cells more than one time, preferably using electroporation. Preferably, RNA is introduced into the cells at least twice, more preferably at least 3 times, more preferably at least 4 times, even more preferably at least 5 times up to preferably 6 times, more preferably up to 7 times or even up to 8, 9 or 10 times, preferably over a time period of at least 10 days, preferably for at least 11 days and more preferably for at least 12 days to ensure expression of one or more factors for an extended period of time. Preferably, the time periods elapsing between the repeated introductions of the RNA are from 24 hours to 120 hours, preferably 48 hours to 96 hours. In one embodiment, time periods elapsing between the repeated introductions of the RNA are not longer than 72 hours, preferably not longer than 48 hours or 36 hours. In one embodiment, prior to the next electroporation, cells are allowed to recover from the previous electroporation. In any case, the conditions should be selected so that the factors are expressed in the cells in amounts and for periods of time which support the reprogramming process.

A "stem cell" is a cell with the ability to self-renew, to remain undifferentiated, and to become differentiated. A stem cell can divide without limit, for at least the lifetime of the animal in which it naturally resides. A stem cell is not terminally differentiated; it is not at the end stage of a differentiation pathway. When a stem cell divides, each daughter cell can either remain a stem cell or embark on a course that leads toward terminal differentiation.

Totipotent stem cells are cells having totipotential differentiation properties and being capable of developing into a complete organism. This property is possessed by cells up to the 8-cell stage after fertilization of the oocyte by the sperm. When these cells are isolated and transplanted into the uterus, they can develop into a complete organism.

Pluripotent stem cells are cells capable of developing into various cells and tissues derived from the ectodermal, mesodermal and endodermal layers. Pluripotent stem cells which are derived from the inner cell mass located inside of blastocysts, generated 4-5 days after fertilization are called "embryonic stem cells" and can differentiate into various other tissue cells but cannot form new living organisms.

Multipotent stem cells are stem cells differentiating normally into only cell types specific to their tissue and organ of origin. Multipotent stem cells are involved not only in the growth and development of various tissues and organs during the fetal, neonatal and adult periods but also in the maintenance of adult tissue homeostasis and the function of inducing regeneration upon tissue damage. Tissue-specific multipotent cells are collectively called "adult stem cells".

An "embryonic stem cell" or "ESC" is a stem cell that is present in or isolated from an embryo. It can be pluripotent, having the capacity to differentiate into each and every cell present in the organism, or multipotent, with the ability to differentiate into more than one cell type.

As used herein, "embryo" refers to an animal in the early stages of it development. These stages are characterized by implantation and gastrulation, where the three germ layers are defined and established and by differentiation of the germs layers into the respective organs and organ systems. The three germ layers are the endoderm, ectoderm and mesoderm.

A "blastocyst" is an embryo at an early stage of development in which the fertilized ovum has undergone cleavage, and a spherical layer of cells surrounding a fluid-filled cavity is forming, or has formed. This spherical layer of cells is the trophectoderm. Inside the trophectoderm is a cluster of cells termed the inner cell mass (ICM). The trophectoderm is the precursor of the placenta, and the ICM is the precursor of the embryo.

An adult stem cell, also called a somatic stem cell, is a stem cell found in an adult. An adult stem cell is found in a differentiated tissue, can renew itself, and can differentiate, with some limitations, to yield specialized cell types of its tissue of origin. Examples include mesenchymal stem cells, hematopoietic stem cells, and neural stem cells.

A "differentiated cell" is a mature cell that has undergone progressive developmental changes to a more specialized form or function. Cell differentiation is the process a cell undergoes as it matures to an overtly specialized cell type. Differentiated cells have distinct characteristics, perform specific functions, and are less likely to divide than their less differentiated counterparts.

An "undifferentiated" cell, for example, an immature, embryonic, or primitive cell, typically has a nonspecific appearance, may perform multiple, non-specific activities, and may perform poorly, if at all, in functions typically performed by differentiated cells.

"Somatic cell" refers to any and all differentiated cells and does not include stem cells, germ cells, or gametes. Preferably, "somatic cell" as used herein refers to a terminally differentiated cell. In one embodiment, the somatic cells are fibroblasts such as lung fibroblasts, foreskin fibroblasts or dermal fibroblasts, or keratinocytes.

In one embodiment, the somatic cells are embryonic stem cell derived somatic cells with a mesenchymal phenotype. In a preferred embodiment, the somatic cells are fibroblasts such as fetal fibroblasts or postnatal fibroblasts or keratinocytes, preferably hair follicle derived keratinocytes. In further embodiments, the fibroblasts are lung fibroblasts, foreskin fibroblasts or dermal fibroblasts. In particular embodiments, the fibroblasts are fibroblasts as deposited at the American Type Culture Collection (ATCC) under Catalog No. CCL-186, as deposited at the American Type Culture Collection (ATCC) under Catalog No. CRL-2097 or as deposited at the American Type Culture Collection (ATCC) under Catalog No. CRL-2522, as distributed by SBI System Biosciences under the catalog no. PC501A-HFF, or as distributed by Innoprot under the catalog no. P10857. In one embodiment, the fibroblasts are adult human dermal fibroblasts. Preferably, the somatic cells are human cells. According to the present teaching, the somatic cells may be genetically modified.

As used herein, "committed" refers to cells which are considered to be permanently committed to a specific function. Committed cells are also referred to as "terminally differentiated cells".

As used herein, "differentiation" refers to the adaptation of cells for a particular form or function. In cells, differentiation leads to a more committed cell.

As used herein, "de-differentiation" refers to loss of specialization in form or function. In cells, de-differentiation leads to a less committed cell.

As used herein "reprogramming" refers to the resetting of the genetic program of a cell. A reprogrammed cell preferably exhibits pluripotency.

The terms "de-differentiated" and "reprogrammed" or similar terms are used interchangeably herein to denote somatic cell-derived cells having stem cell characteristics. However, said terms are not intended to limit the subject-matter disclosed herein by mechanistic or functional considerations.

As used herein, "germ cell" refers to a reproductive cell such as a spermatocyte or an oocyte, or a cell that will develop into a reproductive cell.

As used herein, "pluripotent" refers to cells that can give rise to any cell type except the cells of the placenta or other supporting cells of the uterus.

The term "cells having stem cell characteristics" is used herein to designate cells which, although they are derived from differentiated somatic non-stem cells, exhibit one or more features typical for stem cells, in particular embryonic stem cells. Such features include an embryonic stem cell morphology such as compact colonies, high nucleus to cytoplasm ratio and prominent nucleoli, normal karyotypes, expression of telomerase activity, expression of cell surface markers that are characteristic for embryonic stem cells, and/or expression of genes that are characteristic for embryonic stem cells. The cell surface markers that are characteristic for embryonic stem cells are, for example, selected from the group consisting of stage-specific embryonic antigen-3 (SSEA-3), SSEA-4, tumor-related antigen-1-60 (TRA-1-60), TRA-1-81, and TRA-2-49/6E. The genes that are characteristic for embryonic stem cells are selected, for example, from the group consisting of endogenous OCT4, endogenous NANOG, growth and differentiation factor 3 (GDF3), reduced expression 1 (REX1), fibroblast growth factor 4 (FGF4), embryonic cell-specific gene 1 (ESG1), developmental pluripotency-associated 2 (DPPA2), DPPA4, and telomerase reverse transcriptase (TERT). In one embodiment, the one or more features typical for stem cells include pluripotency. In one embodiment, the cells having stem cell characteristics exhibit a pluripotent state. In one embodiment, the cells having stem cell characteristics have the developmental potential to differentiate into advanced derivatives of all three primary germ layers. In one embodiment, the primary germ layer is endoderm and the advanced derivative is gut-like epithelial tissue. In a further embodiment, the primary germ layer is mesoderm and the advanced derivative is striated muscle and/or cartilage. In an even further embodiment, the primary germ layer is ectoderm and the advanced derivative is neural tissue and/or epidermal tissue. In one preferred embodiment, the cells having stem cell characteristics have the developmental potential to differentiate into neuronal cells and/or cardiac cells. According to the teaching, generally the terms "cells having stem cell characteristics", "cells having stem cell properties", "stem like cells", "reprogrammed cells" and "de-differentiated cells" or similar terms have similar meanings and are used interchangeably herein.

The term "reprogramming factor" according to the teaching includes proteins and peptides as well as derivatives and variants thereof inducing optionally together with further agents such as further reprogramming factors the reprogramming of somatic cells to cells having stem cell characteristics. For example, the term "reprogramming factor" comprises OCT4, SOX2, NANOG, LIN28, KLF4 and c-MYC.

The reprogramming factors can be of any animal species; e.g., mammals and rodents. Examples of mammals include but are not limited to human and non-human primates. Primates include but are not limited to humans, chimpanzees, baboons, cynomolgus monkeys, and any other New or Old World monkeys. Rodents include but are not limited to mouse, rat, guinea pig, hamster and gerbil.

In one embodiment of the present teaching, reprogramming factors capable of allowing the reprogramming of somatic cells to cells having stem cell characteristics comprise an assembly of factors selected from the group consisting of (i) OCT4 and SOX2, (ii) OCT4, SOX2, and one or both of NANOG and LIN28, (iii) OCT4, SOX2 and one or both of KLF4 and c-MYC. In one embodiment, said reprogramming factors comprise OCT4, SOX2, NANOG and LIN28, OCT4, SOX2, KLF4 and c-MYC, or OCT4, SOX2, KLF4, c-MYC, NANOG and LIN28.

OCT4 is a transcription factor of the eukaryotic POU transcription factors and an indicator of pluripotency of embryonic stem cells. It is a maternally expressed Octomer binding protein. It has been observed to be present in oocytes, the inner cell mass of blastocytes and also in the primordial germ cell. The gene *POU5F1* encodes the OCT4 protein. Synonyms to the gene name include *OCT3, OCT4, OTF3* and *MGC22487.* The presence of OCT4 at specific concentrations is necessary for embryonic stem cells to remain undifferentiated. Preferably, "OCT4 protein" or simply "OCT4" relates to human OCT4.

Sox2 is a member of the Sox (SRY-related HMG box) gene family that encode transcription factors with a single HMG DNA-binding domain. SOX2 has been found to control neural progenitor cells by inhibiting their ability to differentiate. The repression of the factor results in delamination from the ventricular zone, which is followed by an exit from the cell cycle. These cells also begin to lose their progenitor character through the loss of progenitor and early neuronal differentiation markers. Preferably, "SOX2 protein" or simply "SOX2" relates to human SOX2.

NANOG is a NK-2 type homeodomain gene, and has been proposed to play a key role in maintaining stem cell pluripotency presumably by regulating the expression of genes critical to embryonic stem cell renewal and differentiation. NANOG behaves as a transcription activator with two unusually strong activation domains embedded in its C terminus. Reduction of NANOG expression induces differentiation of embryonic stem cells. Preferably, "NANOG protein" or simply "NANOG" relates to human NANOG.

LIN28 is a conserved cytoplasmic protein with an unusual pairing of RNA-binding motifs: a cold shock domain and a pair of retroviral-type CCHC zinc fingers. In mammals, it is abundant in diverse types of undifferentiated cells. In pluripotent mammalian cells, LIN28 is observed in RNase-sensitive complexes with Poly(A)-Binding Protein, and in polysomal fractions of sucrose gradients, suggesting it is associated with translating mRNAs. Preferably, "LIN28 protein" or simply "LIN28" relates to human LIN28.

Krueppel-like factor (KLF4) is a zinc-finger transcription factor, which is strongly expressed in postmitotic epithelial cells of different tissues, e.g. the colon, the stomach and the skin. KLF4 is essential for the terminal differentiation of these cells and involved in the cell cycle regulation. Preferably, "KLF4 protein" or simply "KLF4" relates to human KLF4.

MYC (cMYC) is a protooncogene, which is overexpressed in a wide range of human cancers. When it is specifically-mutated, or overexpressed, it increases cell proliferation and functions as an oncogene. MYC gene encodes for a transcription factor that regulates expression of 15% of all genes through binding on Enhancer Box sequences (E-boxes) and recruiting histone acetyltransferases (HATs). MYC belongs to MYC family of transcription factors, which also includes N-MYC and L-MYC genes. MYC-family transcription factors contain the bHLH/LZ (basic Helix-Loop-Helix Leucine Zipper) domain. Preferably, "cMYC protein" or simply "cMYC" relates to human cMYC.

A reference herein to specific factors such as OCT4, SOX2, NANOG, LIN28, KLF4 or c-MYC is to be understood so as to also include all variants of these factors. In particular, it is to be understood so as to also include all splice variants, posttranslationally modified variants, conformations, isoforms and species homologs of these factors which are naturally expressed by cells.

According to the teaching, a variant of a peptide or protein preferably has a functional property of the peptide or protein from which it has been derived. Such functional properties are described herein for OCT4, SOX2, NANOG, LIN28, KLF4 and c-MYC, respectively. Preferably, a variant of a peptide or protein has the same property in reprogramming an animal differentiated cell as the peptide or protein from which it has been derived. Preferably, the variant induces or enhances reprogramming of an animal differentiated cell.

"A functional set of reprogramming factors" is a set of reprogramming factors useful in reprogramming somatic cells to cells having stem cell characteristics, i.e., the set of reprogramming factors when expressed in a somatic cell is sufficient for effecting reprogramming of the somatic cell to a cell having stem cell characteristics.

In one embodiment of the teaching, a functional set of reprogramming factors may comprise more than reprogramming factor which have to be all present within a cell so as to achieve reprogramming. Accordingly, the present teaching involves embodiments wherein a set of RNA molecules such as a set of RNA replicons encodes the different reprogramming factors. For example, different RNA molecules may comprise different open reading frames encoding different reprogramming factors. These different RNA molecules, i.e., a set of RNA molecules, may be co-inoculated into a cell to provide a functional set of reprogramming factors.

The term "miRNA" (microRNA) relates to 21-23-nucleotide-long noncoding RNAs found in eukaryotic cells that, by inducing degradation and/or preventing translation of target mRNAs, modulate a plethora of cell functions, including those related to ESC self-renewal/differentiation and cell cycle progression. miRNAs are post-transcriptional regulators that bind to complementary sequences on target messenger RNA transcripts (mRNAs), usually resulting in translational repression or target degradation and gene silencing. It has been found that miRNAs in the right combination are capable of inducing direct cellular reprogramming of somatic cells to cells having stem cell characteristics *in vitro.* For example, it has been observed that miRNA cluster 302-367 enhances somatic cell reprogramming.

Preferably, the step of allowing the development of cells having stem cell characteristics comprises culturing the somatic cells under embryonic stem cell culture conditions, preferably conditions suitable for maintaining pluripotent stem cells in an undifferentiated state.

Preferably, to allow the development of cells having stem cell characteristics, cells are cultivated in the presence of one or more DNA methyltransferase inhibitors and/or one or more histone deacetylase inhibitors. Preferred compounds are selected from the group consisting of 5'-azacytidine (5'-azaC), suberoylanilide hydroxamic acid (SAHA), dexamethasone, trichostatin A (TSA), sodium butyrate (NaBu), Scriptaid and valproic acid (VPA). Preferably, cells are cultivated in the presence of valproic acid (VPA), preferably in a concentration of between 0.5 and 10 mM, more preferably between 1 and 5 mM, most preferably in a concentration of about 2 mM.

The methods of the present teaching can be used to effect de-differentiation of any type of somatic cell. Cells that may be used include cells that can be de-differentiated or reprogrammed by the methods of the present teaching, in particular cells that are fully or partially differentiated, more preferably terminally differentiated. Preferably, the somatic cell is a diploid cell derived from pre-embryonic, embryonic, fetal, and post-natal multi-cellular organisms. Examples of cells that may be used include but are not limited to fibroblasts, such as fetal and neonatal fibroblasts or adult fibroblasts, keratinocytes, in particular primary keratinocytes, more preferably keratinocytes derived from hair, adipose cells, epithelial cells, epidermal cells, chondrocytes, cumulus cells, neural cells, glial cells, astrocytes, cardiac cells, esophageal cells, muscle cells, melanocytes, hematopoietic cells, osteocytes, macrophages, monocytes, and mononuclear cells. Examples of cells that may be used include blood-derived endothelial cells and endothelial progenitor cells, and urine derived epithelial cells.

The cells with which the methods of the teaching can be used can be of any animal species; e.g., mammals and rodents. Examples of mammalian cells that can be de-differentiated and re-differentiated by the present teaching include but are not limited to human and non-human primate cells. Primate cells with which the teaching may be performed include but are not limited to cells of humans, chimpanzees, baboons, cynomolgus monkeys, and any other New or Old World monkeys. Rodent cells with which the teaching may be performed include but are not limited to mouse, rat, guinea pig, hamster and gerbil cells.

De-differentiated cells prepared according to the present teaching are expected to display many of the same requirements as pluripotent stem cells and can be expanded and maintained under conditions used for embryonic stem cells, e.g. ES cell medium or any medium that supports growth of the embryonic cells. Embryonic stem cells retain their pluripotency *in vitro* when maintained on inactivated fetal fibroblasts such as irradiated mouse embryonic fibroblasts or human fibroblasts (e.g., human foreskin fibroblasts, human skin fibroblasts, human endometrial fibroblasts, human oviductal fibroblasts) in culture. In one embodiment, the human feeder cells may be autologous feeder cells derived from the same culture of reprogrammed cells by direct differentiation.

Furthermore, human embryonic stem cells can successfully be propagated on Matrigel in a medium conditioned by mouse fetal fibroblasts. Human stem cells can be grown in culture for extended period of time and remain undifferentiated under specific culture conditions.

In certain embodiments, the cell culture conditions may include contacting the cells with factors that can inhibit differentiation or otherwise potentiate de-differentiation of cells, e.g., prevent the differentiation of cells into non-ES cells, trophectoderm or other cell types.

De-differentiated cells prepared according to the present teaching can be evaluated by methods including monitoring changes in the cells' phenotype and characterizing their gene and protein expression. Gene expression can be determined by RT-PCR, and translation products can be determined by immunocytochemistry and Western blotting. In particular, de-differentiated cells can be characterized to determine the pattern of gene expression and whether the reprogrammed cells display a pattern of gene expression similar to the expression pattern expected of undifferentiated, pluripotent control cells such as embryonic stem cells using techniques well known in the art including transcriptomics.

The expression of the following genes of de-differentiated cells can be assessed in this respect: OCT4, NANOG, LIN28, growth and differentiation factor 3 (GDF3), reduced expression 1 (REX1), fibroblast growth factor 4 (FGF4), embryonic cell-specific gene 1 (ESG1), developmental pluripotency-associated 2 (DPPA2), DPPA4, telomerase reverse transcriptase (TERT), embryonic antigen-3 (SSEA-3), SSEA-4, tumor-related antigen-1-60 (TRA-1-60), TRA-1-81, and TRA-2-49/6E.

The undifferentiated or embryonic stem cells to which the reprogrammed cells may be compared may be from the same species as the differentiated somatic cells. Alternatively, the undifferentiated or embryonic stem cells to which the reprogrammed cells may be compared may be from a different species as the differentiated somatic cells.

In some embodiments, a similarity in gene expression pattern exists between a reprogrammed cell and an undifferentiated cell, e.g., embryonic stem cell, if certain genes specifically expressed in an undifferentiated cell are also expressed in the reprogrammed cell. For example, certain genes, e.g., telomerase, that are typically undetectable in differentiated somatic cells may be used to monitor the extent of reprogramming. Likewise, for certain genes, the absence of expression may be used to assess the extent of reprogramming.

Self-renewing capacity, marked by induction of telomerase activity, is another characteristic of stem cells that can be monitored in de-differentiated cells.

Karyotypic analysis may be performed by means of chromosome spreads from mitotic cells, spectral karyotyping, assays of telomere length, total genomic hybridization, or other techniques well known in the art.

Using the present teaching, RNA encoding appropriate factors is incorporated into one or more somatic cells, e.g. by electroporation. After incorporation, cells are preferably cultured using conditions that support maintenance of de-differentiated cells (i.e. stem cell culture conditions). The de-differentiated cells can then be expanded and induced to re-differentiate into different type of somatic cells that are needed for cell therapy. De-differentiated cells obtained according to the present teaching can be induced to differentiate into one or more desired somatic cell types *in vitro* or *in vivo.*

Preferably, the de-differentiated cells obtained according to the present teaching may give rise to cells from any of three embryonic germ layers, i.e., endoderm, mesoderm, and ectoderm. For example, the de-differentiated cells may differentiate into skeletal muscle, skeleton, dermis of skin, connective tissue, urogenital system, heart, blood (lymph cells), and spleen (mesoderm); stomach, colon, liver, pancreas, urinary bladder; lining of urethra, epithelial parts of trachea, lungs, pharynx, thyroid, parathyroid, intestine (endoderm); or central nervous system, retina and lens, cranial and sensory, ganglia and nerves, pigment cells, head connective tissue, epidermis, hair, mammary glands (ectoderm). The de-differentiated cells obtained according to the present teaching can be re-differentiated *in vitro* or *in vivo* using techniques known in the art.

In one embodiment of the present teaching, the reprogrammed cells resulting from the methods of this teaching are used to produce differentiated progeny. Thus, in one aspect, the present teaching provides a method for producing differentiated cells, comprising: (i) obtaining reprogrammed cells using the methods of this teaching; and (ii) inducing differentiation of the reprogrammed cells to produce differentiated cells. Step (ii) can be performed *in vivo* or *in vitro.* Furthermore, differentiation can be induced through the presence of appropriate differentiation factors which can either be added or are present in situ, e.g. in a body, organ or tissue into which the reprogrammed cells have been introduced. The differentiated cells can be used to derive cells, tissues and/or organs which are advantageously used in the area of cell, tissue, and/or organ transplantation. If desired, genetic modifications can be introduced, for example, into somatic cells prior to reprogramming. The differentiated cells of the present teaching preferably do not possess the pluripotency of an embryonic stem cell, or an embryonic germ cell, and are, in essence, tissue-specific partially or fully differentiated cells.

One advantage of the methods of the present teaching is that the reprogrammed cells obtained by the present teaching can be differentiated without prior selection or purification or establishment of a cell line. Accordingly in certain embodiments, a heterogeneous population of cells comprising reprogrammed cells are differentiated into a desired cell type. In one embodiment, a mixture of cells obtained from the methods of the present teaching is exposed to one or more differentiation factors and cultured *in vitro.*

Methods of differentiating reprogrammed cells obtained by the methods disclosed herein may comprise a step of permeabilization of the reprogrammed cell. For example, cells generated by the reprogramming techniques described herein, or alternatively a heterogeneous mixture of cells comprising reprogrammed cells, may be permeabilized before exposure to one or more differentiation factors or cell extract or other preparation comprising differentiation factors.

For example, differentiated cells may be obtained by culturing undifferentiated reprogrammed cells in the presence of at least one differentiation factor and selecting differentiated cells from the culture. Selection of differentiated cells may be based on phenotype, such as the expression of certain cell markers present on differentiated cells, or by functional assays (e.g., the ability to perform one or more functions of a particular differentiated cell type).

In another embodiment, the cells reprogrammed according to the present teaching are genetically modified through the addition, deletion, or modification of their DNA sequence(s).

The reprogrammed or de-differentiated cells prepared according to the present teaching or cells derived from the reprogrammed or de-differentiated cells are useful in research and in therapy. Reprogrammed pluripotent cells may be differentiated into any of the cells in the body including, without limitation, skin, cartilage, bone skeletal muscle, cardiac muscle, renal, hepatic, blood and blood forming, vascular precursor and vascular endothelial, pancreatic beta, neurons, glia, retinal, neuronal, intestinal, lung, and liver cells.

The reprogrammed cells are useful for regenerative/reparative therapy and may be transplanted into a patient in need thereof. In one embodiment, the cells are autologous with the patient.

The reprogrammed cells provided in accordance with the present teaching may be used, for example, in therapeutic strategies in the treatment of cardiac, neurological, endocrinological, vascular, retinal, dermatological, muscular-skeletal disorders, and other diseases.

For example, and not intended as a limitation, the reprogrammed cells of the present teaching can be used to replenish cells in animals whose natural cells have been depleted due to age or ablation therapy such as cancer radiotherapy and chemotherapy. In another non-limiting example, the reprogrammed cells of the present teaching are useful in organ regeneration and tissue repair. In one embodiment of the present teaching, reprogrammed cells can be used to reinvigorate damaged muscle tissue including dystrophic muscles and muscles damaged by ischemic events such as myocardial infarcts. In another embodiment of the present teaching, the reprogrammed cells disclosed herein can be used to ameliorate scarring in animals, including humans, following a traumatic injury or surgery. In this embodiment, the reprogrammed cells of the present teaching are administered systemically, such as intravenously, and migrate to the site of the freshly traumatized tissue recruited by circulating cytokines secreted by the damaged cells. In another embodiment of the present teaching, the reprogrammed cells can be administered locally to a treatment site in need of repair or regeneration.

The agents described herein such as nucleic acids, in particular RNA, and cells, in particular when used for the treatments described herein, may be present in the form of a pharmaceutical composition or kit comprising the agent and optionally one or more pharmaceutically acceptable carriers, diluents and/or excipients.

Pharmaceutical compositions are preferably sterile and contain an effective amount of the nucleic acid.

Pharmaceutical compositions are usually provided in a uniform dosage form and may be prepared in a manner known in the art. The pharmaceutical composition may, e.g., be in the form of a solution or suspension.

The pharmaceutical composition may comprise salts, buffer substances, preservatives, carriers, diluents and/or excipients all of which are preferably pharmaceutically acceptable. The term "pharmaceutically acceptable" refers to the non-toxicity of a material which does not interfere with the action of the active component(s) of the pharmaceutical composition.

Salts which are not pharmaceutically acceptable may be used for preparing pharmaceutically acceptable salts and are included in the teaching. Pharmaceutically acceptable salts of this kind comprise, in a non-limiting way, those prepared from the following acids: hydrochloric, hydrobromic, sulfuric, nitric, phosphoric, maleic, acetic, salicylic, citric, formic, malonic, succinic acids, and the like. Pharmaceutically acceptable salts may also be prepared as alkali metal salts or alkaline earth metal salts, such as sodium salts, potassium salts or calcium salts.

Suitable buffer substances for use in the pharmaceutical composition include acetic acid in a salt, citric acid in a salt, boric acid in a salt and phosphoric acid in a salt.

Suitable preservatives for use in the pharmaceutical composition include benzalkonium chloride, chlorobutanol, paraben and thimerosal.

The term "carrier" refers to an organic or inorganic component, of a natural or non-natural (synthetic) nature, with which the active component is combined in order to facilitate, enhance or enable application. According to the teaching, the term "carrier" also includes one or more compatible solid or liquid fillers, diluents or encapsulating substances, which are suitable for administration to a patient.

Possible carrier substances for parenteral administration are, e.g., sterile water, glucose solutions, Ringer, Ringer lactate, sterile sodium chloride solution, polyalkylene glycols, hydrogenated naphthalenes and, in particular, biocompatible lactide polymers, lactide/glycolide copolymers or polyoxyethylene/polyoxy-propylene copolymers.

The term "excipient" when used herein is intended to indicate all substances which may be present in a pharmaceutical composition and which are not active ingredients such as, e.g., carriers, binders, lubricants, thickeners, surface active agents, preservatives, emulsifiers, buffers, flavoring agents, or colorants.

The pharmaceutical compositions described herein may be administered via any conventional route, such as by parenteral administration including by injection or infusion. Administration is preferably parenterally, e.g. intravenously, intraarterially, subcutaneously, in the lymph node, intradermally or intramuscularly.

Compositions suitable for parenteral administration usually comprise a sterile aqueous or non-aqueous preparation of the active compound, which is preferably isotonic to the blood of the recipient. Examples of compatible carriers and solvents are Ringer's solution and isotonic sodium chloride solution. In addition, usually sterile, fixed oils are used as solution or suspension medium.

The agents and compositions described herein are preferably administered in effective amounts. An "effective amount" refers to the amount which achieves a desired reaction or a desired effect alone or together with further doses. In the case of treatment of a particular disease or of a particular condition, the desired reaction preferably relates to inhibition of the course of the disease. This comprises slowing down the progress of the disease and, in particular, interrupting or reversing the progress of the disease. The desired reaction in a treatment of a disease or of a condition may also be delay of the onset or a prevention of the onset of said disease or said condition.

An effective amount of an agent or composition described herein will depend on the condition to be treated, the severeness of the disease, the individual parameters of the patient, including age, physiological condition, size and weight, the duration of treatment, the type of an accompanying therapy (if present), the specific route of administration and similar factors. Accordingly, the doses administered of the agents described herein may depend on several of these parameters. In the case that a reaction in a patient is insufficient with an initial dose, higher doses (or effectively higher doses achieved by a different, more localized route of administration) may be used.

The present invention is described in detail by the following figures and examples.

### Figures

Figure 1: NSs can replace EKB for inhibition of IFN-response to synthetic mRNA in human fibroblasts
Figure 2: NSs enables RNA-based Reprogramming
Figure 3: NSs inhibits IFN-response to SFV-self-replicating RNA and prevents protein kinase R activation
Figure 4: NSs increases transfection rates with VEEV self-replicating RNA, increases translation and blocks IFN-response
Figure 5: NSs improves the expression of VEEV trans-replicons and inhibits IFN-response
Figure 6: NSs improves transreplicon expression in immune cells

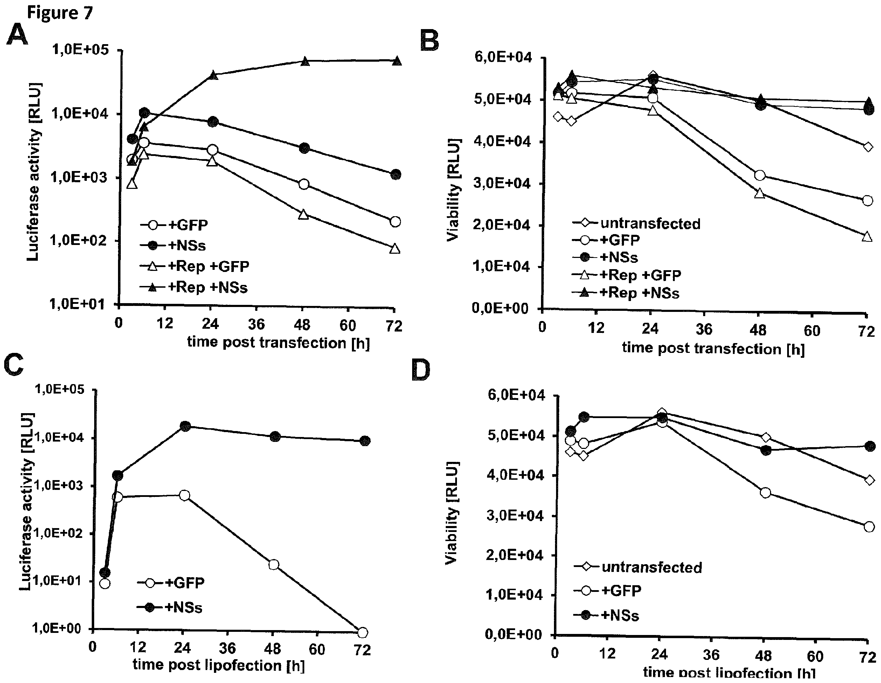

Figure 7: NSs improves expression of VEEV trans-replicating and self-replicating RNA in immune cells and prevents cell death

### Examples

### Example 1: NSs can replace EKB for inhibition of IFN-response to synthetic mRNA in human fibroblasts

The generation of induced pluripotent stem (iPS) cells holds great promises to liberate stem cells research from ethical concerns associated with the use of human embryonic stem (hES) cells. Continuous expression of exogenous pluripotency-associated transcription factors (TF) until the pluripotency is maintained by the endogenous pluripotency network is thereby the key to achieve reprogramming of somatic cells. In regard to clinical translation of the iPS-technology it is crucial to circumvent any risk of genomic integration in the targeted cells genome. Synthetic mRNA is therefore probably the most suitable vector since synthetic mRNA is strictly transient and complex RNA mixtures can be easily transfected into somatic cells. Nevertheless synthetic mRNA is recognized by cellular receptors that can distinguish between specific molecular patterns distinct from cellular RNAs. This leads to defense mechanisms of the cell resulting in apoptosis, cytoskeletal rearrangements, RNA degradation and a shutdown of translation which hinders successful reprogramming of cells. We were able to establish an non-modified RNA-based reprogramming approach in which the viral escape proteins E3, K3 and B18R (EKB) are used to counteract the IFN-response of the cell allowing successful reprogramming of human fibroblasts and blood-outgrowth endothelial progenitor cells (EPCs) (Hum Gene Ther. 2015 Nov;26(11):751-66). The viral escape protein NSs from *Toscana virus* was shown to be a potent inhibitor of IFN-response. Here we investigate the effect of NSs on the IFN-response of human fibroblasts. In a first step NSs effect on reporter expression (GFP), reduction of IFN-response genes (OAS1/IFNβ) and cell survival after daily lipofections was analyzed in comparison to the gold standard EKB.

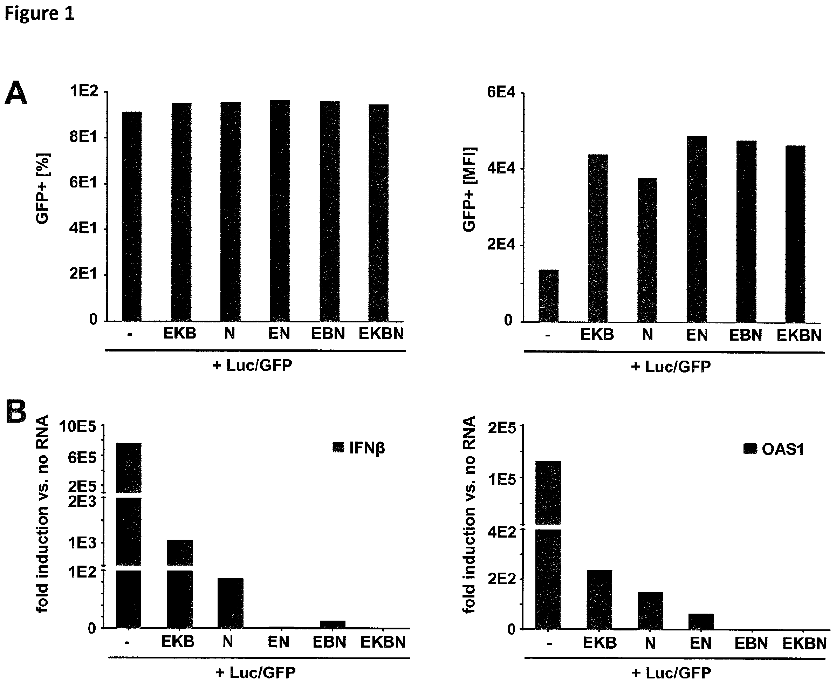

Primary human foreskin fibroblasts (HFFs, System Bioscience) were plated into 6 wells (100,000 cells/well) and lipofected the next day using 6µl RNAiMAX (Invitrogen) and 1.4 µg non-modified synthetic mRNA (*in vitro* transcribed). The synthetic mRNA mixtures were thereby composed of 0.8 µg green florescent protein (GFP) together with different mixtures of IFN-escape proteins E3 (E), K3 (K), B18R (B) and / or NSs (N) as indicated in Figure 1A. 0.2 µg synthetic mRNA of each IFN-escape protein was used and synthetic mRNA encoding Luciferase (Luc) was utilized to sum up the mixtures to 1.4 µg. Lipofections were performed according to the manufacturer's instructions. Cells were harvested 24h post transfection and half of the cells were used for analysis of GFP expression by FACS. It became obvious that all samples were lipofected equally with a transfection efficiency of 91-96% (Figure 1A, left panel). We observed a ~3-fold increase of GFP mRNA translation when co-transfected with EKB. This increase was slightly lower when N was used alone and slightly higher when co-transfected with EN. Additional IFN-escape proteins (K and B) did not further increase translation of GFP mRNA.

The rest of cells from the previous experiment was pelleted, total RNA was isolated and mRNA-expression of IFNβ and OAS1 was quantified by qRT-PCR (Figure 1B). As expected, EKB is able to downregulate induction of IFNβ and OAS1 expression by synthetic mRNA when co-transfected. This could also be achieved with N alone, even better in case of IFNβ induction. N in combination with other viral escape proteins (EKB) reduced IFN-response of the cell to synthetic mRNA to a minimum.

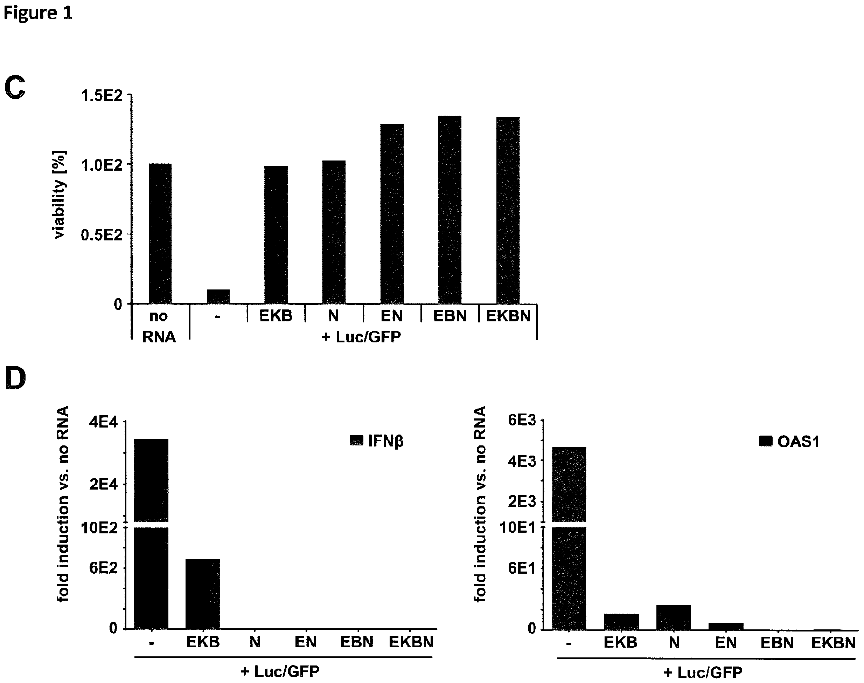

HFFs were plated into Swells (100,000 cells/well) and lipofected the next three consecutive days using 6µl RNAiMAX (Invitrogen) and 1.4 µg synthetic mRNA. The synthetic mRNA mixtures were thereby composed of 0.8 µg GFP together with different mixtures of IFN-escape proteins E3 (E), K3 (K), B18R (B) and / or NSs (N) as indicated in Figure 1C. 0.2 µg synthetic mRNA of each IFN-escape protein was used and synthetic mRNA encoding Luciferase (Luc) was utilized to sum up the mixtures to 1.4 µg. Lipofections were performed according to the manufacturer's instructions. 24h after the last lipofection, cell viability was assayed using the Cell Proliferation Kit II (Roche) with normalization to mock transfected cells. As expected cells die when they are repetitive transfected with synthetic mRNA. This can be rescued by co-transfection with EKB. Unexpectedly, N alone was also able do enhance survival of cells and could therefore replace EKB. If combined with E3 there was also a slight proliferative effect observed.

Cells from the previous experiment were pelleted 24h after the last transfection, total RNA was isolated and mRNA-expression of IFNβ and OAS1 was quantified by qRT-PCR (Figure 1D). In accordance to the survival analysis in Figure 1C, EKB reduces induction of IFNβ and OAS1 by synthetic mRNA significantly. This could also be achieved when EKB is replaced by N alone. If N is combined with other viral escape proteins, the IFN-response of the cells is nearly extinguished.

The data shows that NSs can replace EKB in inhibition of IFN-response to synthetic mRNA in human fibroblasts. NSs alone is able to counteract a downregulation of translation, it inhibits the upregulation of IFN-response genes and prevents loss of cell viability.

### Example 2: NSs enables RNA-based Reprogramming

As shown in Example 1, NSs can replace EKB in inhibition of IFN-response to synthetic mRNA in human fibroblasts. As stated before, we previously developed an RNA-based reprogramming approach in which the viral escape proteins E3, K3 and B18R (EKB) are used to counteract the IFN-response of the cell allowing successful reprogramming of human fibroblasts and blood-outgrowth endothelial progenitor cells (EPCs) into human induced pluripotent stem (iPS) cells (Hum Gene Ther. 2015 Nov;26(11):751-66). Here we investigate whether NSs alone is also able to facilitate RNA-based reprogramming of human fibroblasts.

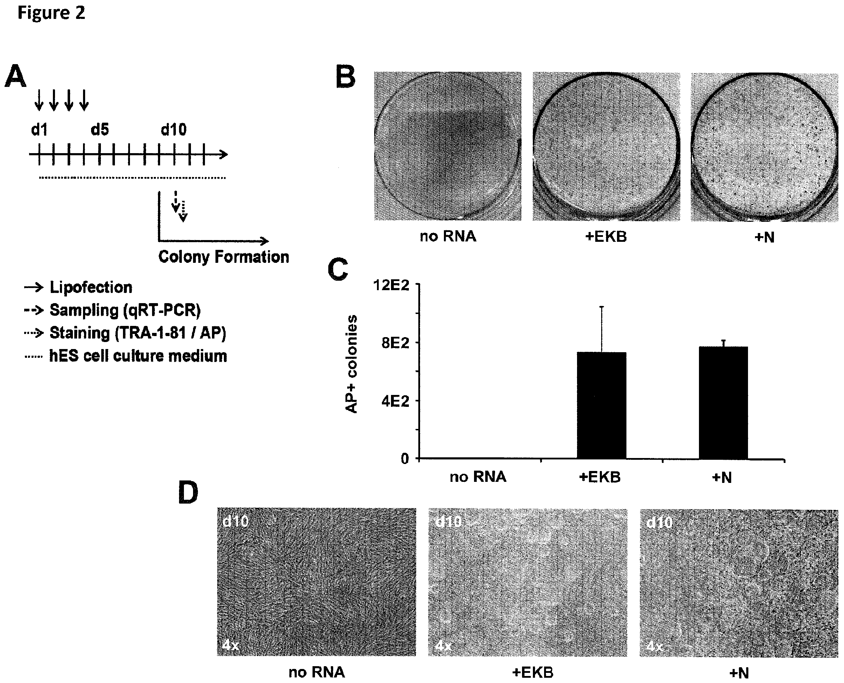

Figure 2A shows the timeline for the reprogramming of primary human dermal fibroblasts (HDFs, Innoprot). 40,000 cells were plated into a 12-well-plate and after 4 hours of cultivation lipofected on four consecutive days with synthetic mRNA mixtures that were composed of 0.33 µg non-modified synthetic mRNA encoding the reprogramming transcription factors (TF) OCT4, SOX2, KLF4, cMYC, NANOG and LIN28 (OsSKMNL) (3:1:1:1:1:1) with 0.08 µg of each B18R, E3 and K3 (EKB) or 0.24 µg of NSs (N) alone and in both cases with 0.17 µg of a miRNA mixture composed of miRNAs 302a-d and 367 (1:1:1:1:1). Cells were cultivated in human embryonic stem (hES) cell medium (NutriStem media, Stemgent) and lipofections using 2.5 µL RNAiMAX (Life Technologies) per 12well were performed according to the manufacturer's instructions. From day 9 on, colony formation was observed and analysis of colonies were performed on d10.

Established colonies were stained for alkaline phosphatase (AP) on day 10 using an AP Staining Kit (SBI) and following the manufacturers instructions. For an overview, representative stainings are shown Figure 2B. It became obvious that formation of AP positive colonies (red/dark) is quite similar when EKB or N alone is used.

AP positive colonies from two independent experiments were counted and are shown in Figure 2C as mean colony count ± SD (n=2). The data confirms the results from the overview: RNA-based reprogramming of human fibroblasts into iPS cells can be performed with EKB or NSs alone with similar reprogramming efficiencies.

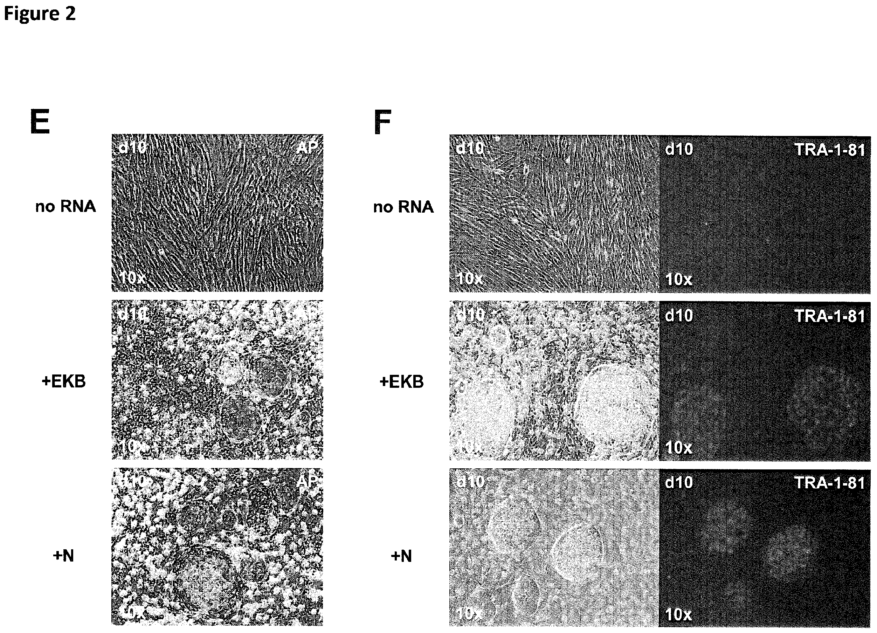

Colony morphology of resulting iPS-cell colonies when using NSs alone instead of EKB were similar hES cell-like with tightly packed small cells in distinct colonies and well-defined borders (Figure 2D). These colonies could as well be stained positive for AP (Figure 2E) (red/dark) and the hES cell surface marker TRA-1-81 (Figure 2F) (green/white). TRA-1-81 live staining was performed with the Stain-Alive TRA-1-81 antibody (Stemgent) according to the manufacturer's instructions. Representative pictures of colonies are shown.

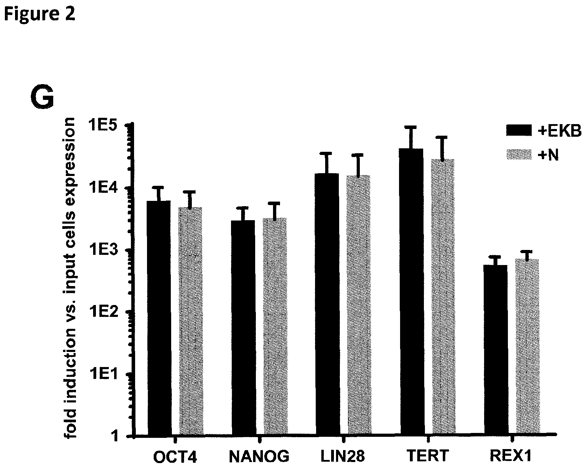

To further assess pluripotency of colonies, cells were pelleted, total RNA isolated and mRNA-expression of the hES-markers OCT4 (endogenous), NANOG (endogenous), LIN28 (endogenous), TERT and REX1 was quantified by qRT-PCR. mRNA expression was normalized to that of HPRT and is shown as mean fold induction ± SD (n=2) compared to the transcript levels of input cells. All analyzed markers were highly expressed compared to input cells indicating pluripotency of reprogrammed cells. Again there was no significant difference observed when NSs alone instead of EKB was used to facilitate RNA-based reprogramming of human fibroblasts (Figure 2G).

NSs is able to reduce the IFN-response of human fibroblasts to synthetic mRNA to a similar degree then the use of the three viral escape proteins E3, K3 and B18R. This allows multiple transfections of an RNA-based reprogramming cocktail leading to the successful reprogramming of human fibroblasts into human iPS cells. Successful reprogramming of cells using NSs alone instead of EKB was thereby confirmed by hES-cell like morphology, AP-activity and the expression of hES-cell surface and endogenous markers of resulting iPS-cell colonies. Reprogramming efficiency and quality of reprogramming using NSs was shown to be as good as EKB enabled RNA-based reprogramming.

### Example 3: NSs inhibits IFN-response to SFV-self-replicating RNA and prevents protein kinase R activation

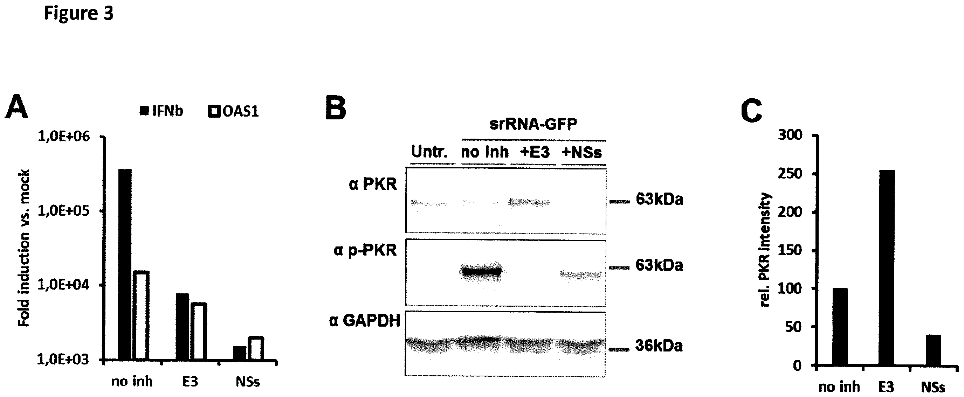

Human foreskin fibroblasts were plated into 6-well-plates and transfected the next day. To this aim 2.5µg GFP encoding self-replicating RNA (viral origin Semliki Forest Virus), or 1.25µg self-replicating RNA together with 1.25µg mRNA encoding either Vaccinia virus E3 or Toscana virus NSs were complexed with MessengerMax and added to the cells. Cells were harvested 17h after transfection and used to determine interferon response by qPCR (upregulation of IFNβ and OAS1) and PKR-degradation and activation by immunoblotting against total PKR and autophosphorylated PKR (p-PKR). NSs resulted in a more pronounced reduction of IFN response than E3 (Figure 3A). NSs overexpression reduced PKR expression compared to untransfected (untr.) cells, cells transfected with replicon alone, or replicon+E3. However, it does not prevent activation of residual PKR (Figure 3B). Figure 3C shows a densitometric quantification of the band intensities of the PKR blot.

### Example 4: NSs increases transfection rates with VEEV self-replicating RNA, increases translation and blocks IFN-response

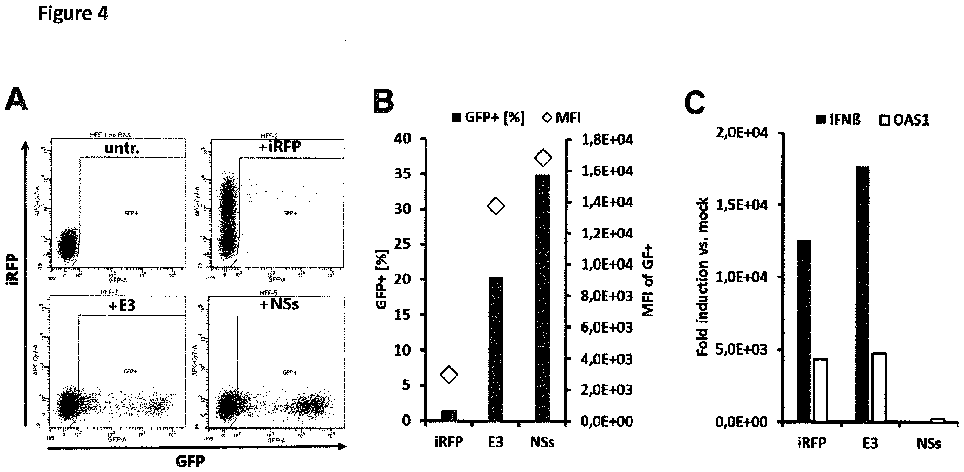

Human foreskin fibroblasts were plated into 6-well-plates and transfected the next day. To this aim 1.11Jg GFP encoding self-replicating RNA (viral origin Venezuelan equine encephalitis virus) and 1.4µg mRNA encoding either infrared fluorescent protein (iRFP) or Vaccinia virus E3 or Toscana virus NSs were complexed with MessengerMax and added to the cells. 24h later, cells were harvested to determine transfection rates by flow cytometry and interferon response by qPCR (upregulation of IFNb and OAS1). Flow cytometric analysis shows an increased transfection rate upon cotransfection of E3 and NSs (untr. = untransfected cells) (Figure 4A,B). Cotransfer of NSs resulted in a more pronounced reduction of IFN response than cotransfer of E3 (Figure 4C).

### Example 5: NSs improves the expression of VEEV trans-replicons and inhibits IFN-response

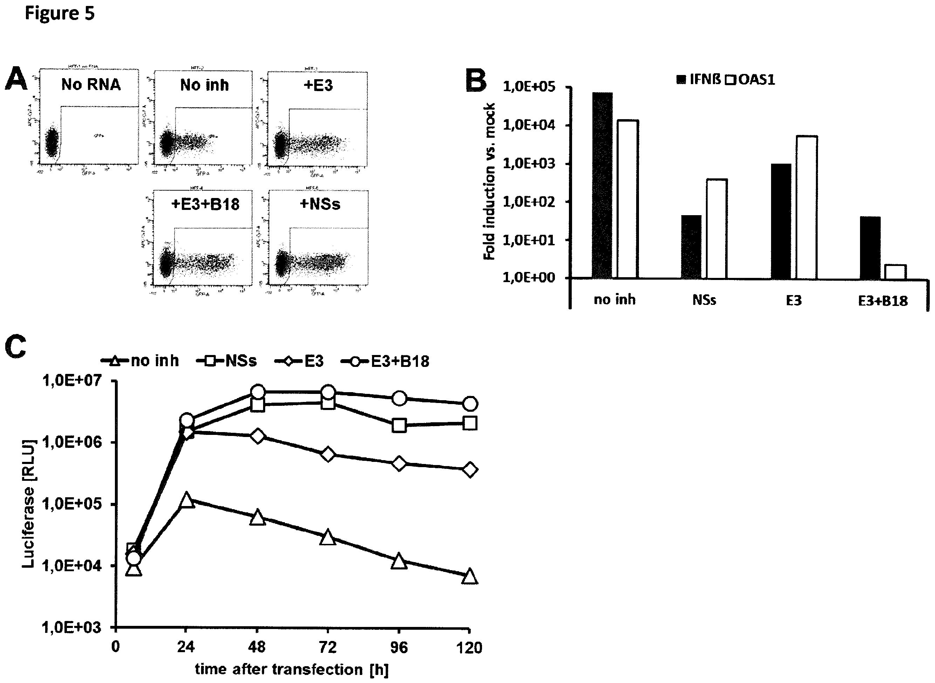

Human foreskin fibroblasts were electroporated with 1.4µg mRNA encoding VEEV replicase, 0.3µg transreplicating RNA encoding GFP and 0.3µg transreplicating RNA encoding luciferase. To inhibit interferon response the samples were supplemented with 2µg NSs mRNA, E3 mRNA, or E3 and B18 mRNA (1µg each). 24h later cells were harvested to analyze GFP expression by flow cytometry (Figure 5A), and interferon response by qPCR (upregulation of IFNb and OAS1) (Figure 5B). Furthermore, luciferase expression was measured for 5 days at the time points indicated in Figure 5C.

NSs improves expression as good as the combination of E3 and B18, and inhibits IFN response comparably.

### Example 6: NSs improves transreplicon expression in immune cells

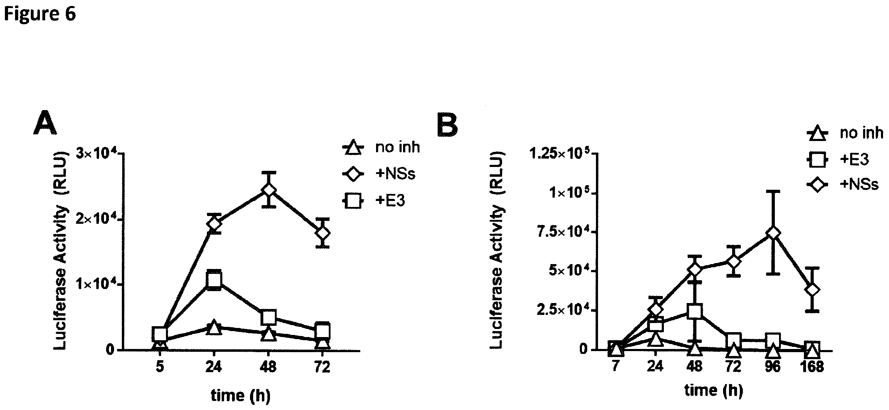

Resting peripheral CD8 positive human T cells were electroporated with 5µg mRNA encoding VEEV replicase, and 5µg transreplicating RNA encoding luciferase. To inhibit interferon response the samples were supplemented with 5µg NSs mRNA or E3 mRNA. Luciferase expression was assessed at the time points indicated in Figure 6A.

Human peripheral immature dendritic cells were electroporated with 1.4µg mRNA encoding VEEV replicase, 0.3µg transreplicating RNA encoding luciferase and 0.3µg transreplicating RNA encoding GFP. To inhibit interferon response the samples were supplemented with 2µg NSs mRNA or E3 mRNA. Luciferase expression was assessed at the time points indicated in Figure 6B.

### Example 7: NSs improves expression of VEEV trans-replicating and self-replicating RNA in immune cells and prevents cell death

**(A)** Human peripheral immature dendritic cells were lipofected with MessengerMax (Invitrogen) in 96-well-plates with 25 ng total RNA per well. 3 ng transreplicating RNA encoding luciferase were co-transfected or not with 12 ng mRNA encoding VEEV replicase (+Rep), and 10 ng mRNA encoding GFP (+GFP) or the inhibitor NSs (+NSs) as indicated. Luciferase expression was assessed at the indicated time points (BrightGlo Assay; Promega). **(B)** Viability of the transfected cells was also assessed (CellTiterGlo assay; Promega) and plotted against viability of untransfected cells. **(C, D)** In parallel, cells were also transfected with 10 ng VEEV-replicon encoding luciferase, and either GFP or NSs. Luciferase expression (C) and viability (D) were followed for 72h after lipofection.

In dendritic cells, NSs prolongs expression of self- and trans-replicating RNA and inhibits cell death.

## Claims

1. An *in vitro* method for expressing one or more chains of a T cell receptor or of an artificial T cell receptor in a T-cell or in an antigen presenting cell, the method comprising the steps of (i) introducing RNA encoding one or more chains of a T cell receptor or of an artificial T cell receptor into the cell and (ii) providing *Toscana virus* NSs protein to the cell, wherein providing *Toscana virus* NSs protein to the cell comprises introducing RNA encoding the *Toscana virus* NSs protein into the cell.

2. The method of claim 1, wherein the RNA is introduced into the cell repetitively.

3. The method of claim 1 or 2, wherein the RNA is introduced into the cell by electroporation or lipofection.

4. The method of any one of claims 1 to 3, wherein the RNA is *in vitro* transcribed RNA.

5. The method of any one of claims 1 to 4, further comprising providing vaccinia virus B18R and/or vaccinia virus E3 to the cell, wherein providing vaccinia virus B18R and/or vaccinia virus E3 to the cell comprises introducing RNA encoding vaccinia virus B18R and/or RNA encoding vaccinia virus E3 into the cell.

6. The method of any one of claims 1 to 5, wherein the cell is a human cell.

7. An *in vitro* method of producing immunoreactive cells comprising the step of (i) introducing into a T cell or a progenitor thereof one or more RNA molecules encoding the chains of a T cell receptor or the chain(s) of an artificial T cell receptor, and (ii) providing Toscana virus NSs protein to the cell,
wherein providing *Toscana virus* NSs protein to the cell comprises introducing RNA encoding the *Toscana virus* NSs protein into the cell.

8. The method of claim 7, wherein immunoreactive cells comprise T cells such as cytotoxic T cells, helper T cells, or tumor infiltrating T cells.

9. The method of claim 8, wherein immunoreactive cells comprise CD4+ and/or CD8+ T cells.

10. The method of claim 7, wherein immunoreactive cells comprise cells which can mature into immune cells, such as T cell, in particular T helper cell, or cytolytic T cell, with suitable stimulation.

11. The method of claim 7, wherein immunoreactive cells comprise CD34+ hematopoietic stem cells, immature and mature T cells.

12. The method of any one of claims 7 to 11, wherein the T cell receptor comprises α- and β-TCR chains.

13. The method of any one of claims 7 to 12, wherein the artificial T cell receptor comprises an antigen binding domain such as antigen-binding portions of antibodies and T cell receptors such as single-chain variable fragments (scFv), a T cell signaling domain, such as the endodomain of CD3-zeta, and optionally one or more co-stimulation domains, such as CD28, CD137 (4-1BB), CD134 (OX40), and CD278 (ICOS).

## Patentansprüche

1. *In vitro*-Verfahren zur Expression einer oder mehrerer Ketten eines T-Zell-Rezeptors oder eines künstlichen T-Zell-Rezeptors in einer T-Zelle oder in einer Antigen-präsentierenden Zelle, wobei das Verfahren die Schritte umfasst: (i) Einbringen von RNA, die für eine oder mehrere Ketten eines T-Zell-Rezeptors oder eines künstlichen T-Zell-Rezeptors kodiert, in die Zelle und (ii) Bereitstellen von *Toscana*-*Virus*-NSs-Protein für die Zelle, wobei das Bereitstellen des *Toscana*-*Virus*-NSs-Proteins für die Zelle das Einbringen von RNA, die für das *Toscana-Virus*-NSs-Protein kodiert, in die Zelle umfasst.

2. Verfahren nach Anspruch 1, wobei die RNA wiederholt in die Zelle eingebracht wird.

3. Verfahren nach Anspruch 1 oder 2, wobei die RNA durch Elektroporation oder Lipofektion in die Zelle eingebracht wird.

4. Verfahren nach einem der Ansprüche 1 bis 3, wobei die RNA in *vitro*-transkribierte RNA ist.

5. Verfahren nach einem der Ansprüche 1 bis 4, das ferner das Bereitstellen von Vaccinia-Virus-B18R und/oder Vaccinia-Virus-E3 für die Zelle umfasst, wobei das Bereitstellen von Vaccinia-Virus-B18R und/oder Vaccinia-Virus-E3 für die Zelle das Einbringen von RNA, die für Vaccinia-Virus-B18R kodiert, und/oder RNA, die für Vaccinia-Virus-E3 kodiert, in die Zelle umfasst.

6. Verfahren nach einem der Ansprüche 1 bis 5, wobei die Zelle eine menschliche Zelle ist.

7. *In vitro*-Verfahren zur Herstellung immunreaktiver Zellen, das den Schritt des (i) Einbringens eines oder mehrerer RNA-Moleküle, die für die Ketten eines T-Zell-Rezeptors oder die Kette(n) eines künstlichen T-Zell-Rezeptors kodieren, in eine T-Zelle oder einen Vorläufer davon und (ii) Bereitstellens von *Toscana*-*Virus*-NSs-Protein für die Zelle umfasst, wobei das Bereitstellen von *Toscana*-*Virus*-NSs-Protein für die Zelle das Einbringen von RNA, die das *Toscana*-*Virus*-NSs-Protein kodiert, in die Zelle umfasst.

8. Verfahren nach Anspruch 7, wobei immunreaktive Zellen T-Zellen, wie zytotoxische T-Zellen, Helfer-T-Zellen oder tumorinfiltrierende T-Zellen, umfassen.

9. Verfahren nach Anspruch 8, wobei immunreaktive Zellen CD4+- und/oder CD8+-T-Zellen umfassen.

10. Verfahren nach Anspruch 7, wobei immunreaktive Zellen Zellen umfassen, die zu Immunzellen, wie T-Zellen, insbesondere T-Helferzellen, oder zytolytische T-Zellen, bei geeigneter Stimulation reifen können.

11. Verfahren nach Anspruch 7, wobei immunreaktive Zellen CD34+-hämatopoetische Stammzellen, unreife und reife T-Zellen umfassen.

12. Verfahren nach einem der Ansprüche 7 bis 11, wobei der T-Zell-Rezeptor α- und β-TCR-Ketten umfasst.

13. Verfahren nach einem der Ansprüche 7 bis 12, wobei der künstliche T-Zell-Rezeptor eine Antigenbindungsdomäne, wie antigenbindende Teile von Antikörpern und T-Zell-Rezeptoren, wie einzelkettige variable Fragmente (scFv), eine T-Zell-Signaldomäne, wie die Endodomäne von CD3-zeta, und gegebenenfalls eine oder mehrere Co-Stimulationsdomänen, wie CD28, CD137 (4-1BB), CD134 (OX40) und CD278 (ICOS), umfasst.

## Revendications

1. Méthode *in vitro* permettant l'expression d'une ou plusieurs chaînes d'un récepteur de lymphocytes T ou d'un récepteur de lymphocytes T artificiel dans un lymphocyte T ou dans une cellule présentatrice d'antigène, la méthode comprenant les étapes de (i) introduction d'ARN codant une ou plusieurs chaînes d'un récepteur de lymphocytes T ou d'un récepteur de lymphocytes T artificiel dans la cellule et (ii) fourniture d'une protéine NSs de *Toscana virus* à la cellule, ladite fourniture d'une protéine NSs de *Toscana virus* à la cellule comprenant l'introduction d'ARN codant la protéine NSs de *Toscana virus* dans la cellule.

2. Méthode selon la revendication 1, ledit ARN étant introduit dans la cellule de manière répétitive.

3. Méthode selon la revendication 1 ou 2, ledit ARN étant introduit dans la cellule par électroporation ou lipofection.

4. Méthode selon l'une quelconque des revendications 1 à 3, ledit ARN étant un ARN transcrit *in vitro.*

5. Méthode selon l'une quelconque des revendications 1 à 4, comprenant en outre la fourniture de la B18R de virus de la vaccine et/ou de l'E3 de virus de la vaccine à la cellule, ladite fourniture de la B18R de virus de la vaccine et/ou de l'E3 de virus de la vaccine à la cellule comprenant l'introduction d'ARN codant la B 18R de virus de la vaccine et/ou d'ARN codant l'E3 de virus de la vaccine dans la cellule.

6. Méthode selon l'une quelconque des revendications 1 à 5, ladite cellule étant une cellule humaine.

7. Méthode *in vitro* de production de cellules immunoréactives comprenant l'étape de
(i) introduction dans un lymphocyte T ou un progéniteur de celle-ci d'une ou plusieurs molécules d'ARN codant les chaînes d'un récepteur de lymphocytes T ou la ou les chaînes d'un récepteur de lymphocytes T artificiel, et (ii) fourniture d'une protéine NSs de *Toscana virus* à la cellule,
ladite fourniture d'une protéine NSs de *Toscana virus* à la cellule comprenant l'introduction d'ARN codant la protéine NSs de *Toscana virus* dans la cellule.

8. Méthode selon la revendication 7, lesdites cellules immunoréactives comprenant les lymphocytes T tels que les lymphocytes T cytotoxiques, les lymphocytes T auxiliaires ou les lymphocytes T infiltrant les tumeurs.

9. Méthode selon la revendication 8, lesdites cellules immunoréactives comprenant les lymphocytes T CD4+ et/ou CD8+.

10. Méthode selon la revendication 7, lesdites cellules immunoréactives comprenant les cellules qui peuvent se développer en cellules immunitaires, tel qu'un lymphocyte T, en particulier un lymphocyte T auxiliaire, ou un lymphocyte T cytotoxique, avec une stimulation appropriée.

11. Méthode selon la revendication 7, lesdites cellules immunoréactives comprenant les cellules souches hématopoïétiques CD34+, les lymphocytes T matures et immatures.

12. Méthode selon l'une quelconque des revendications 7 à 11, ledit récepteur de lymphocytes T comprenant des chaînes de α- et β-TCR.

13. Méthode selon l'une quelconque des revendications 7 à 12, ledit récepteur de lymphocytes T artificiel comprenant un domaine de liaison à un antigène tel que des parties de liaison à un antigène d'anticorps et les récepteurs de lymphocytes T tels que les fragments variables à chaîne unique (scFv), un domaine de signalisation de lymphocytes T, tel que l'endodomaine de CD3-zéta, et éventuellement un ou plusieurs domaines de co-stimulation, tels que CD28, CD137 (4-1BB), CD134 (OX40) et CD278 (ICOS).
